# EUROPÄISCHE PATENTANMELDUNG

(11) **EP 2 679 225 A1**
(43) Veröffentlichungstag der Anmeldung: **01.01.2014**
(21) Anmeldenummer: 12173548.4
(22) Anmeldetag: 26.06.2012
(51) Int. Cl.: A61K 31/35, A61P 9/00, A61P 9/04, A61P 9/12, A61K 31/19, A61K 31/375, A61K 39/395, A61P 35/00, A61K 31/198, A61K 31/337, A61K 31/352, A61K 31/055, A61K 31/454, A61K 31/55

(54) **Pharmazeutische Zusammensetzung mit synergistischer Wirkung von direkten Katalaseinhibitoren und zur Katalasezerstörung führenden Modulatoren des NO-Stoffwechsels oder der extrazellulären Superoxidanionenproduktion**

(71) Anmelder: Universitätsklinikum Freiburg, 79106 Freiburg (DE)
(72) Erfinder: Bauer, Georg, 79104 Freiburg (DE)
(74) Vertreter: Keller, Günter

(57) **Zusammenfassung**

Die vorliegende Patentanmeldung betrifft eine pharmazeutische Zusammensetzung, dadurch gekennzeichnet, dass sie wenigstens einen Wirkstoff enthält, der eine Singulettsauerstoff-unabhängige direkte Katalase-Inaktivierung bewirken kann und, dass die Zusammensetzung weiterhin mindestens einen Wirkstoff enthält, der durch Modulation des Stickoxid oder Superoxidanionenstoffwechsels der Zellen und nachfolgender Singulettsauerstoffbildung zur Inaktivierung von Katalase führt.

## Beschreibung

Die vorliegende Erfindung beruht auf dem unerwarteten Befund, dass die Kombination von direkten Hemmstoffen der Katalase mit Modulatoren des NO- oder Superoxidanionenstoffwechsels, die zunächst Singulettsauerstoff bilden und nachfolgend Katalase irreversibel inaktivieren, zu einem starken synergistischen Effekt bei der selektiven Apoptoseinduktion in Tumorzellen führt. Dabei kann durch die Kombination von jeweils wenigstens einem Wirkstoff aus der Gruppe der direkten Katalasehemmstoffe mit jeweils wenigstens einem Wirkstoff der Gruppe der Modulatoren des NO- oder Superoxidanionenstoffwechsels deutliche und selektive Apoptoseinduktion in Tumorzellen erreicht werden, obwohl die Anwendung der jeweiligen Wirkstoffe allein im gleichen Konzentrationsbereich zu keiner erkennbaren Apoptoseinduktion führt.

Dieser Befund ist deshalb unerwartet, weil aufgrund des bisherigen Wissenstandes angenommen werden musste, dass sich die Wirkung von direkten Katalasehemmstoffen und solchen Wirkstoffen, die über die Modulation des NO- oder Superoxidanionenstoffwechsels zu einer Singulettsauerstoff-abhängigen Katalasezerstörung führen in ihrer Wirkung lediglich additiv ergänzen könnten.

Auf der Basis dieser Befunde wird eine spezifische Form der synergistischen Antitumorwirkung mittels Apoptoseinduktion offenbart.

### Begriffsdefinitionen:

A) **Direkte Katalaseinhibitoren:** Wirkstoffe, die unmittelbar zu einer Hemmung der Katalase, bevorzugt der außen auf der Membran von Tumorzellen befindlichen protektiven Katalase führen. Für die Wirkung dieser Gruppe von Katalasehemmstoffen ist keine Bildung von Singulettsauerstoff notwendig. Als Konsequenz der Katalasehemmung ergibt sich selektive Apoptoseinduktion in Tumorzellen, die durch interzelluläres ROS-Signaling gesteuert und bewirkt wird. Zu den "direkten Katalasehemmstoffen" in diesem Sinne werden neben Antikörpern gegen Katalase im erweiterten Sinn auch Antikörper gegen SOD gezählt, da es nach Hemmung der Tumorzell-SOD aufgrund des Anstiegs der freien Superoxidanionenkonzentration zwangsläufig zu einer Hemmung von Katalase kommt. An diesem Prozess ist kein Singulettsauerstoff beteiligt. Bevorzugte Beispiele für "direkte Katalaseinhibitoren" sind: 3-Aminotriazol, Antikörper gegen Katalase und gegen SOD, Salicylsäure, Ascorbinsäure und Methyl-Dopa. Direkte Katalaseinhibition mit Relevanz für Apoptoseinduktion in Tumorzellen kann bestimmt werden, indem Tumorzellen zunächst in Gegenwart des Singulettsauerstofffängers Histidin (2 mM) mit dem zu testenden Stoff in steigender Konzentration vorinkubiert werden (zwischen 10 und 30 Minuten bei 37°C) und anschließend Peroxynitrit im Konzentrationsbereich zwischen 10 und 200 µM zugegeben wird. Die Tumorzellen sind durch ihre membranständige Katalase vor der Apoptoseauslösenden Wirkung von Peroxynitrit geschützt. Eine Hemmung der Katalase hebt diesen Schutz teilweise oder ganz auf und führt in Gegenwart von Peroxynitrit zur Apoptoseinduktion. Durch die Anwesenheit von 2mM Histidin wird sichergestellt, dass es sich bei der gemessenen Apoptoseinduktion nicht um eine Singulettsauerstoff-vermittelte Inaktivierung der Katalase durch Zerstörung ihres aktiven Zentrums handelt. Alternativ kann statt Histidinzugabe die Hemmung der NADPH-Oxidase durch 100 µM AEBSF erfolgen. Bei unterbundener Superoxidanionenproduktion ist ebenfalls keine Singulettsauerstoffbildung im Testsystem möglich.
B) **Modulatoren des NO- oder Superoxidanionenstoffwechsels, die zu einer Inaktivierung der Katalase führen:** Diese Gruppe von Wirkstoffen führt durch eine Erhöhung der verfügbaren NO-Konzentration oder durch Steigerung der Superoxidanionenproduktion über mehrere biochemisch definierte Schritte zur Bildung von Singulettsauerstoff, der durch Reaktion mit dem im aktiven Zentrum der Katalase befindlichen Histidin die Katalase inaktiviert und dadurch interzelluläres ROS-Signaling ermöglicht. Beispiele für Modulatoren des NO-Stoffwechsels sind Arginin, Arginasehemmer wie z. B. Nor-NOHA, Hemmstoffe der NO-Dioxygenase (NOD) wie z. B. Flavonoide, Taxol, Epothilon B, Anthocyane, Artemisinin, Azole, Diallyldisulfid, Palmitinsäure und Induktoren der NOS-Expression wie z. B. Interferon Y. Beispiele für Modulatoren der Superoxidanionproduktion durch Stimulation von NOX-1 sind Resveratrol, Transforming Growth Factor-beta.

### Hintergrund der vorliegenden Erfindung

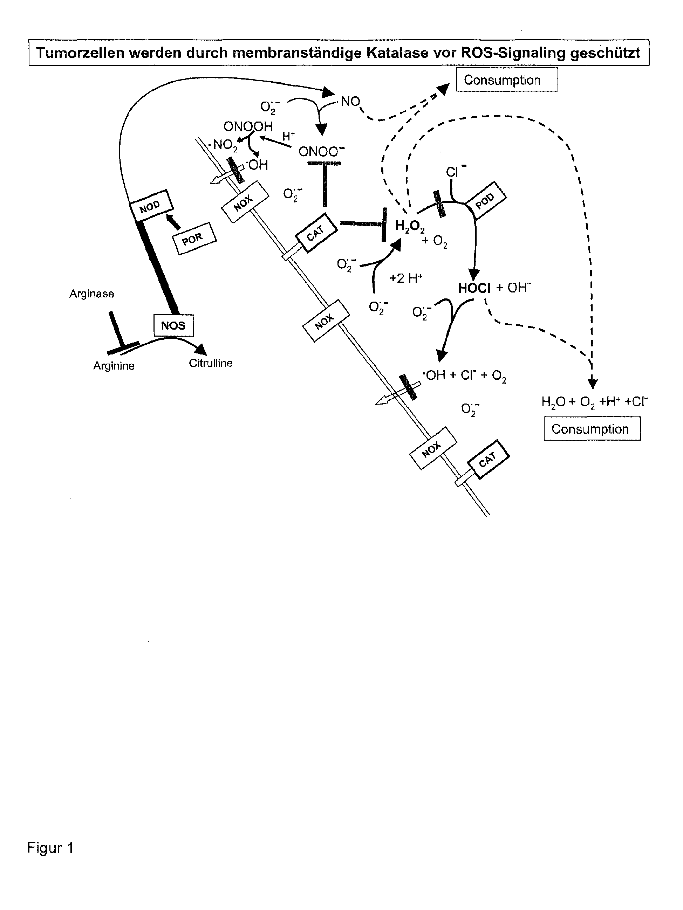

Maligne Zellen sind durch die extrazelluläre Produktion von Superoxidanionen durch membranständige NADPH-Oxidase (NOX-1) charakterisiert. Dabei wird die Aktivität der NOX durch Onkogene wie z. B. RAS unter Einschaltung von RAC gesteuert. Die durch maligne Zellen generierten Superoxidanionen und deren Dismutationsprodukt Wasserstoffperoxid sind für die Proliferation dieser Zellen und für die Aufrechterhaltung ihres transformierten Zustandes essentiell (zusammengefasst in Heinzelmann and Bauer, [2010], Biol. Chem. Vol. 391, pp. 675-693). Die Kehrseite der Medaille aus der extrazellulären Produktion von Reaktiven Sauerstoffspezies (ROS) ist jedoch die Entstehung von interzellulären ROS-vermittelten Signalwegen, die sich selektiv gegen Zellen mit dem transformierten Phänotyp richten. Es sind dies die in Figur 1 gezeigten Hauptwege, nämlich der HOCI- und der NO/Peroxynitritsignalweg, sowie zwei weitere Wege von untergeordneter Bedeutung, nämlich der Nitrylchloridweg und die Metall-katalysierte Haber-Weiss-Reaktion, die in der Figur 1 nicht berücksichtigt werden. Im Verlauf der Tumorprogression erwerben Tumorzellen Resistenz gegen die interzellulären ROS-Signalwege, indem sie auf ihrer Zellmembran Katalase (CAT) exprimieren. Diese hemmt den HOCI- und den Nitrylchloridweg, sowie die Metall-katalysierte Haber-Weiss-Reaktion, indem sie Wasserstoffperoxid in Wasser und Sauerstoff umsetzt, und wirkt dem NO/Peroxynitritweg entgegen, indem sie Peroxynitrit zersetzt und mithilfe ihrer aktiven Zwischenstufe CAT Fe^{IV} = O⁺(Compound I) NO in NO₂ oxidiert und dadurch die Bildung von Peroxynitrit verhindert.

Die Aufhebung des durch Katalase (CAT) vermittelten Schutzes von Tumorzellen stellt ein attraktives Konzept für die Entwicklung einer neuärtigen Form der Tumortherapie dar, die sich spezifisch gegen Zellen mit dem malignen Phänotyp (aufgrund der Merkmale membranständige Katalase und Superoxidanionenproduktion) richtet und normale Zellen nicht gefährdet, da diese weder extrazelluläre Superoxidanionen produzieren noch membranständig Katalase exprimieren.

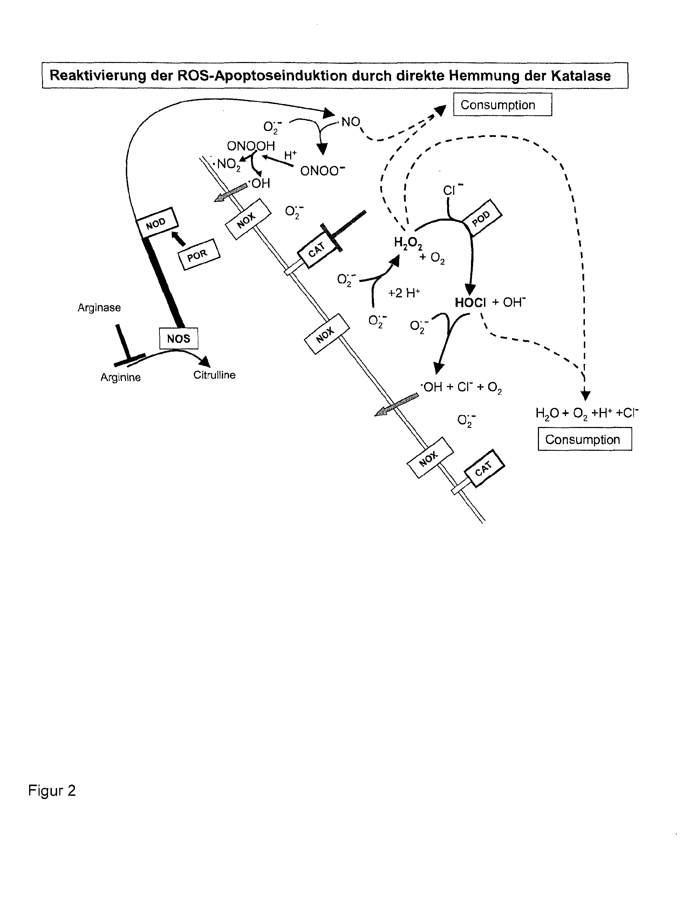

Die in DE 103 58 077 A1 2005.07.28 offenbarte Vorgehensweise erlaubt die Suche nach Wirkstoffen, die gezielt und direkt die membranständige Katalase hemmen und dadurch die interzellulären ROS-Signalwege reaktivieren, die dann in den Tumorzellen Apoptose induzieren. Figur 2 zeigt schematisch die ROS (reactive oxygen species) gesteuerte Apoptoseinduktion, die durch die Hemmung der Katalase reaktiviert wird.

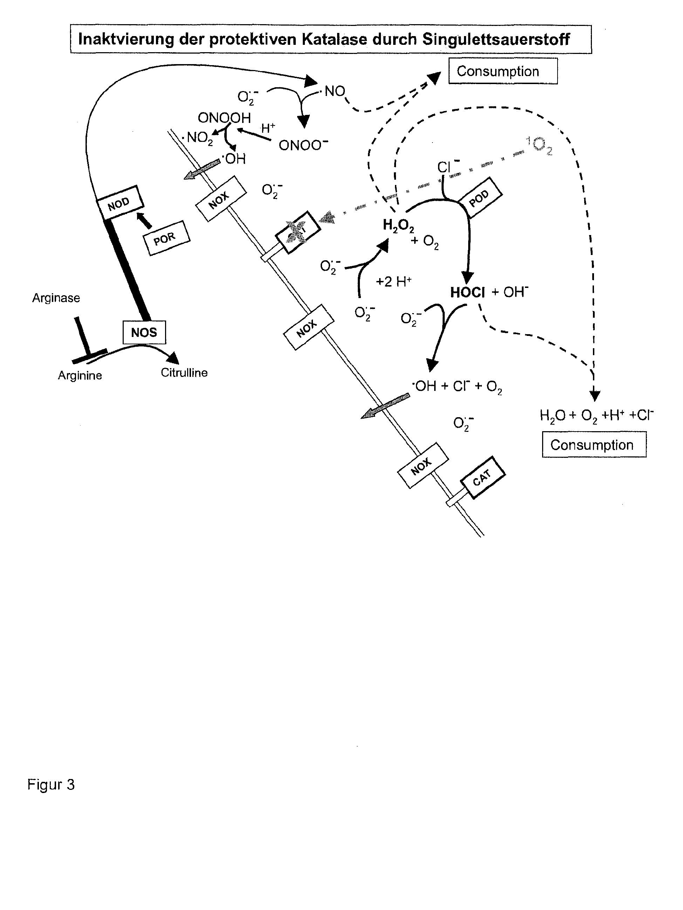

Die gleiche selektive Apoptoseinduktion in Tumorzellen kann erreicht werden, wenn extrazellulär Singulettsauerstoff gebildet wird (zum Beispiel durch die Belichtung des Photosensitizers Photofrin), der dann mit einem Histidinrest in der Katalase reagiert und diese so inaktiviert. Dies ist schematisch in Figur 3 dargestellt.

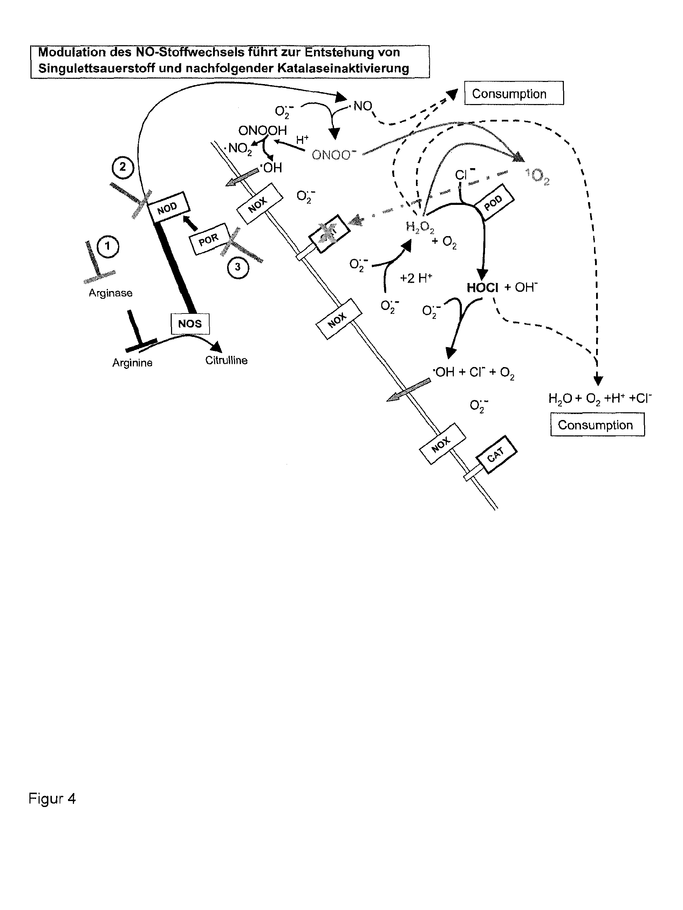

Die Bildung von Singulettsauerstoff kann jedoch auch durch Modulation des NO-Stoffwechsels der Zellen erreicht werden. Dies ist schematisch in Figur 4 dargestellt. Hemmung zellulärer Arginase ①, Zugabe von Arginin, Hemmung der NO-Dioxygenase (NOD) ② oder der damit assoziierten Cytochrom P450-abhängigen Oxidoreduktase (POR) ③ führen zu einem sprunghaften Anstieg der verfügbaren NO-Konzentration. Daraus resultiert über komplexe Amplifikationsschritte schließlich die Bildung von extrazellulärem Singulettsauerstoff, der die Katalase inaktiviert. In der EPA 07.870201.6 wird ein Verfahren zur Identifizierung von Verbindungen offenbart, die Tumor-Apoptose durch Inaktivieren von Katalase auf der Oberfläche von Tumorzellen induzieren.

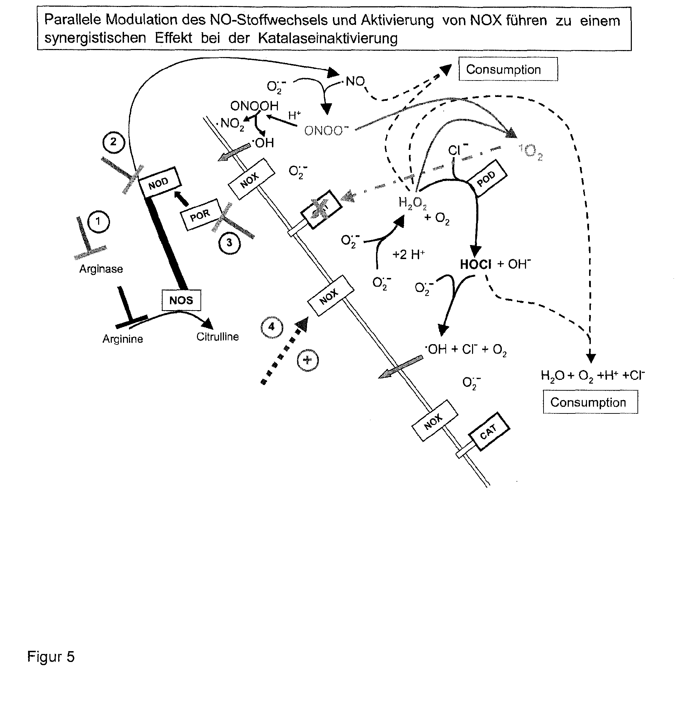

Weiter wurde beschrieben, dass sich durch die Manipulation des NO-Stoffwechsels und die gleichzeitige Verstärkung der NOX-Aktivität ein bemerkenswerter synergistischer Effekt erzielen lässt, der zu einer Absenkung der benötigten Konzentrationen der Einzelwirksubstanzen führt. Dieser Wirkmechanismus ist in der Figur 5 schematisch dargestellt. Die Nutzung des synergistischen Effektes zwischen Modulatoren des NO- und der NADPH-Oxidase ist in der europäischen Patentanmeldung Nr. 10.173500.9 offenbart.

Während die oben erläuterten Synergiemechanismen stets an verschiedenen Teilstrecken der ROS-Signalwege ansetzten, betrifft die hier vorgelegte Erfindung die synergistische Interaktion zwischen einer oder zwei Teilstrecken des ROS-Signaling und einer subtoxischen Hemmung der Katalase. Diese Kombinationen führen dann insgesamt zu einer massiven Inaktivierung der Katalase und zum nachfolgenden selektiven Zelltod der Tumorzellen.

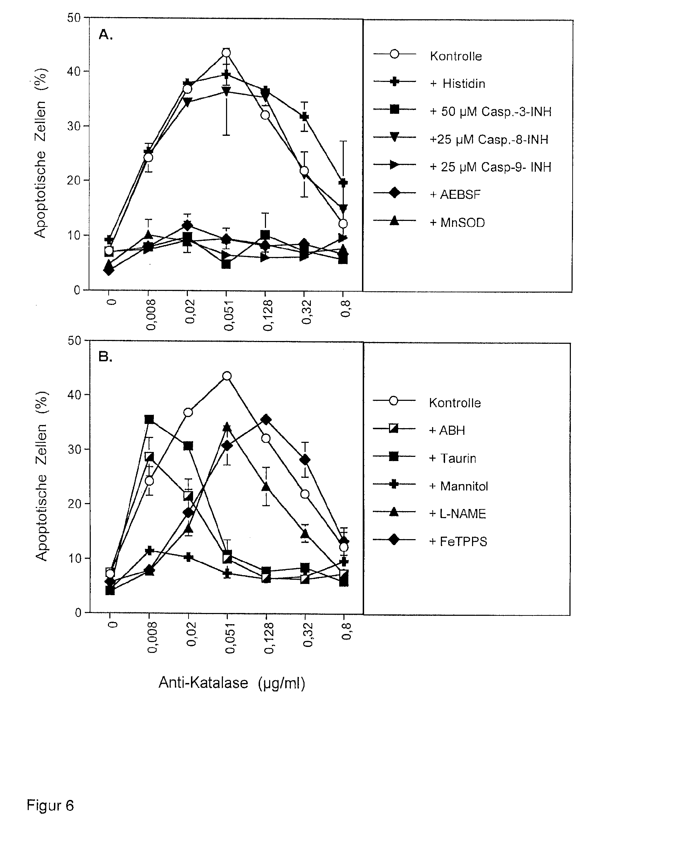

Die experimentellen Ergebnisse, die zu der vorliegenden Erfindung geführt haben, sind nachfolgend in Form der Figuren 6 und folgende, sowie der detaillierten Legende zu diesen Figuren offenbart.

### Liste der in den Experimenten bzw. Figuren verwendeten Abkürzungen

- AEBSF: 4-(2-Aminoethyl)-benzenesulfonyl fluoride (Hemmstoff der NADPH-Oxidase)
- ABH: 4-Aminobenzoyl hydrazid (Peroxidasehemmstoff)
- 3-AT: 3-Aminotriazol (Katalasehemmstoff)
- aCAT: Antikörper gegen Katalase; bevorzugt verwendet wurde ein monoklonaler Antikörper (Maus; IgG1) gegen humane Katalase (Clone CAT-505) (Chargennummer: 088K4809) Hersteller Sigma Aldrich, Schnelldorf, Deutschland.
- aSOD: Antikörper gegen SOD; bevorzugt verwendet wurde ein monoklonaler Antikörper (Maus, IgG1) gegen humane SOD-1 (Clone SD-G6) (Chargennummer 035K4823). Hersteller Sigma Aldrich, Schnelldorf, Deutschland.
- CAT: Katalase
- Compound I: Aktivierte Zwischenstufe der Katalase mit der Formel CAT Fe^{IV} = O⁺. Compound I wird bei der Reaktion von Katalase mit einem Molekül Wasserstoffperoxid oder Peroxynitrit gebildet.
- DEA NONOate: 2-(N,N-Diethylamino)-diazenolate-2-oxide diethylammonium Salz (schnell zerfallender NO-Donor)
- Duox: Duale Oxidase (membranständiges Enzym das aus einer NADPH-Oxidase- und einer Peroxidasedomäne besteht. Die Peroxidasedomäne wird mithilfe von Proteasen abgespalten)
- FBS: Fetale Bovine Serum (fötales Kälberserum)
- FeTPPS: 5-, 10-, 15-, 20-Tetrakis(4-sulfonatophenyl)porphyrinato iron(III) chloride (Peroxynitrit Zersetzungskatalysator)
- MnTE-2PyP: Mn(III) meso-tetrakis(N-ethyl-2-pyridyl)porphyrin Pentachloride (zellgängiges SOD-Mimetikum)
- L-NAME: N-ω-nitro-L-arginine methylester hydrochloride (NOS-Inhibitor)
- NO: Nitric oxide (Stickstoffmonoxid)
- NOD: Nitric oxide dioxygenase (oxidiert NO zu Nitrat)
- NOS: NO-Synthase
- NOX: NADPH-oxidase (hier insbesondere die membranständige NOX-1)
- POD: Peroxidase; hier kommt insbesondere die Fähigkeit bestimmter Peroxidasen zum Tragen, die in Gegenwart von Wasserstoffperoxid Chlorid zu HOCI oxidieren können.
- PON: Peroxynitrit
- POR: Cytochrom P 450 Oxidoreduktase
- RAS, RAC: Onkogene
- ROS: Reactive oxygen and nitrogen species; radikalische und nichtradikalische Spezies, wie Superoxidanionen, Hydroxylradikale, Stickstoffmonoxid, Wasserstoffperoxid, HOCI, Peroxynitrit, etc.
- siRNA: small interfering RNA; werden verwendet als Reagenz zum spezifischen Herunterregulieren der Synthese definierter Genprodukte
- SOD: Superoxiddismutase, bevorzugt SOD-1 (Cu⁺⁺ im aktiven Zentrum der Tumorzellen und MnSOD aus Bakterien für analytische Zwecke)
- TGF-beta: Transforming growth factor type beta

Gegenstand der vorliegenden Erfindung sind pharmazeutische Zusammensetzungen, die dadurch gekennzeichnet sind, dass sie einerseits wenigstens einen Wirkstoff enthalten, der eine Singulettsauerstoff-unabhängige direkte Katalase-Inaktivierung bewirken kann. Weiterhin enthalten diese Zusammensetzungen mindestens einen weiteren Wirkstoff, der durch Modulation des Stickoxid- oder Superoxidanionenstoffwechsels der Zellen und nachfolgender Singulettsauerstoffbildung zur Inaktivierung von Katalase führt.

In bevorzugter Ausführungsform handelt es sich bei dem Wirkstoff, der zu Singulettsauerstoff-unabhängiger direkter Inaktivierung der Katalase fähig ist, um Antikörper gegen Katalase sowie um Antikörper gegen Superoxiddismutase. Die Herstellung derartiger Antikörper ist dem Fachmann bekannt. Bevorzugt handelt es sich hierbei um monoklonale, humanisierte oder humane Antikörper, die spezifisch an Epitope der menschlichen Katalase oder der menschlichen Superoxiddismutase binden, die sich bevorzugt auf den Oberflächen von Zellen, insbesondere von Tumorzellen befinden. Andere Beispiele von geeigneten Wirkstoffen sind Salicylsäure und davon abgeleitete Derivate, Ascorbinsäure und Methyl-dopa.

Bei dem anderen Wirkstoff, der in den erfindungsgemäßen pharmazeutischen Zusammensetzungen vorhanden ist, handelt es sich um Wirkstoffe, die durch Modulation des Stickoxid- oder Superoxidanionenstoffwechsels der Zellen und nachfolgender Singulettsauerstoffbildung zur Inaktivierung von Katalase führen. Diese Wirkstoffe sind bevorzugt ausgewählt aus der Stoffgruppe, die die verfügbare Stickoxidkonzentration in Zellen erhöhen kann. In besonders bevorzugter Ausführungsform handelt es sich bei dem Wirkstoff, der durch Modulation des Stickoxid- oder Superoxidanionenstoffwechsels der Zellen und nachfolgender Singulettsauerstoffbildung zur Inaktivierung von Katalase führt, um Verbindungen, die ausgewählt sind aus Verbindungen der Gruppen Flavonoide, Anthocyane, Taxol, Epothilon B, Artemisin, Diallyldisulfid, Nor-NOHA, 2(S)-Amino-6-boronohexanoic acid.NH₄ (A-6-BHA), (2S)-(+)-Amino-5-iodoacetamidopentanoic acid (A-5-IAAPA), S-(2-Boronoethyl)-L-cysteine.NH₄ (BEC), N^{G}-Hydroxy-L-arginine.monoacetate ("NOHA"), Azole (wie z. B. Itraconazol, Econazol, Fluconazol, Mikonazol, Ketokonazol), Isothiocyanate wie z. B. Methylisothiocyanat, Allylisothiocyanat, und Sulforaphan.

In bevorzugter Ausführungsform beinhaltet eine erfindungsgemäße pharmazeutische Zusammensetzung wenigstens einen Wirkstoff, der eine Singulettsauerstoff-unabhängige direkte Katalase-Inaktivierung bewirken kann, wobei dieser ausgewählt ist aus humanen oder humanisierten Antikörpern gegen membranständige humane Katalase oder humanen oder humanisierten Antikörpern gegen humane Superoxiddismutase. Der Wirkstoff, der durch Modulation des Stickoxid- oder Superoxidanionenstoffwechsels der Zellen und nachfolgender Singulettsauerstoffbildung zur Inaktivierung der Katalase führt, ist ausgewählt aus Taxol, einem Anthocyan oder Diallyldisulfid.

In einer besonders bevorzugten Ausführungsform umfasst die pharmazeutische Zusammensetzung einen humanen oder humanisierten Antikörper gegen membranständige humane Katalase und Taxol.

In einer weiteren besonders bevorzugten Ausführungsform umfasst die erfindungsgemäße pharmazeutische Zusammensetzung einen humanen oder humanisierten Antikörper gegen membranständige humane Katalase und Anthocyan.

In einer weiteren bevorzugten Ausführungsform umfasst die erfindungsgemäße pharmazeutische Zusammensetzung einen humanen oder humanisierten Antikörper gegen membranständige humane Katalase und Diallyldisulfid.

In einer weiteren bevorzugten Ausführungsform können die erfindungsgemäßen pharmazeutischen Zusammensetzungen weiterhin Resveratrol enthalten.

Die jeweiligen Wirkstoffe werden in den erfindungsgemäßen pharmazeutischen Zusammensetzungen in solchen Konzentrationen eingesetzt, dass eine ausreichende Konzentration an den jeweiligen Tumorzellen bewirkt werden kann. Die aktuell eingesetzten Konzentrationen hängen davon ab, ob es sich um pharmazeutische Zusammensetzungen handelt, die oral verabreicht werden, oder um Zusammensetzungen, die parenteral verabreicht werden, wie Injektionslösungen, Infusionslösungen und ähnliches.

Die erfindungsgemäßen pharmazeutischen Zusammensetzungen werden bevorzugt verwendet zur Behandlung von Tumorerkrankungen. Bei den Tumorerkrankungen, die mit den erfindungsgemäßen Zusammensetzungen behandelt werden können, handelt es sich bevorzugt um Tumorformen, die unter *in vivo*-Bedingungen die zur Katalasezerstörung notwendigen Signalmoleküle in ausreichender Konzentration zur Verfügung stellen. Bevorzugt handelt es sich um Tumorerkrankungen, die ausgewählt sind aus Magenkarzinom oder Lymphom.

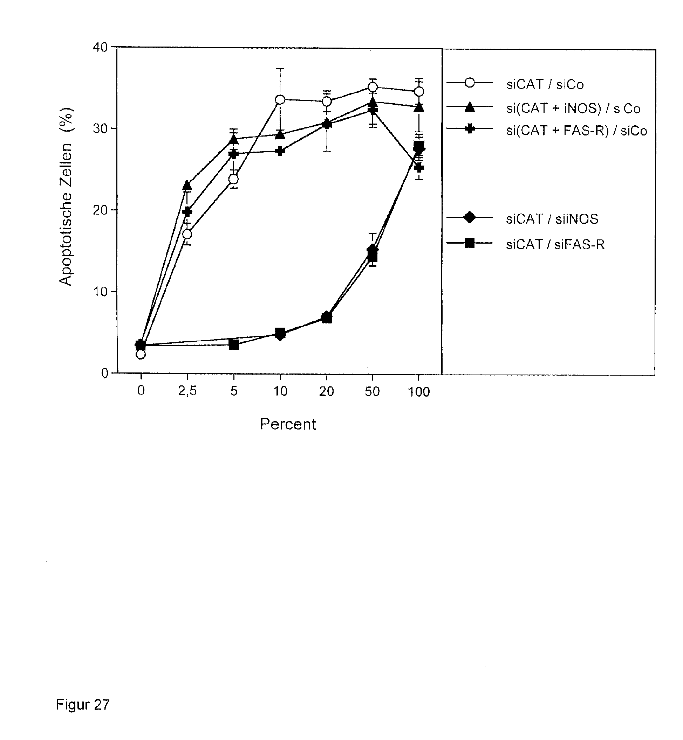

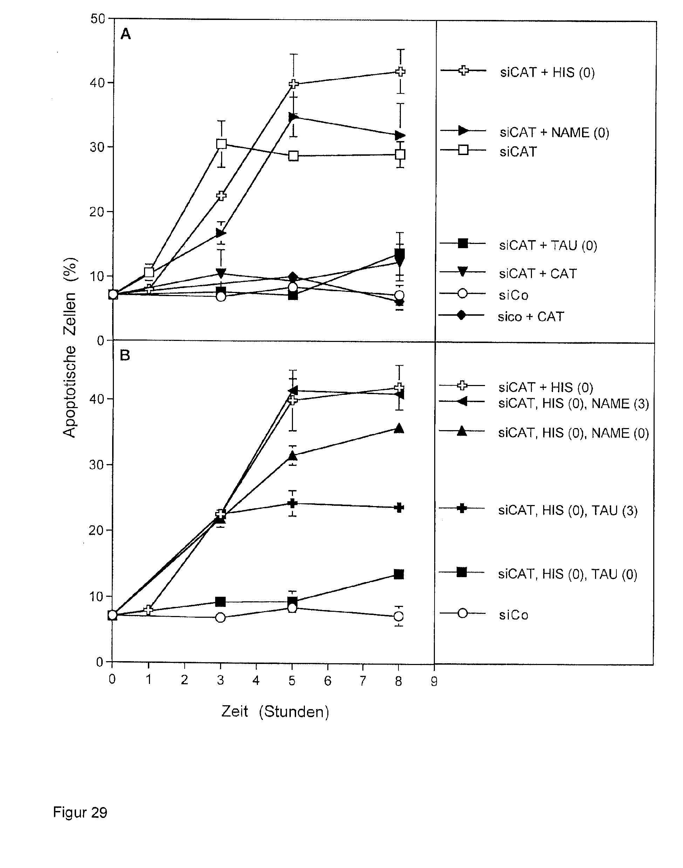

Die nachfolgende Erläuterung der Figuren beschreibt die vorliegende Erfindung. Die Figuren 1-5 geben die Interaktionen der einzelnen Komponenten wieder, die der vorliegenden Erfindung zugrundeliegen. Weiterhin wurden Versuche durchgeführt und die Ergebnisse der Experimente wurden in den Figuren 6-29 wiedergegeben.

### Figur 1

### Tumorzellen sind durch membranständige Katalase vor interzellulärem ROS-Signaling geschützt

Die Abbildung zeigt links den interzellulären, rechts den intrazellulären Bereich einer Tumorzelle und dazwischen das potenzielle interzelluläre ROS-Signaling. In der Zellmembran befindet sich die aktivierte NADPH-Oxidase NOX-1, die extrazellulär Superoxidanionen generiert. Diese dismutieren spontan zu Wasserstoffperoxid und Sauerstoff. Die Zellen setzen Peroxidase (POD) frei, die von DUOX kodiert und durch Matrixmetalloproteasen abgespalten wird. Wasserstoffperoxid und Chloridionen werden von der freien Peroxidase (POD) zu HOCI umgesetzt, welches mit Superoxidanionen zu Apoptose-induzierenden Hydroxylradikalen reagiert. Bei relativem Überschuss an Wasserstoffperoxid kommt es zur Verbrauchsreaktion von HOCI, die den HOCI-Weg schwächt. NO-Synthase (NOS) generiert NO, welches entweder von Wasserstoffperoxid in einer komplexen Konsumreaktion verbraucht wird oder mit Superoxidanionen zu Peroxynitrit reagiert. Nach Bildung von Peroxynitritsäure und deren Zerfall in Hydroxylradikale und NO₂ kommt es zur Apoptoseinduktion. Die beiden untergeordneten Signalwege Nitrylchloridweg und Metall-katalysierte Haber-Weiss-Reaktion sind in dem Schema nicht berücksichtigt.

Es wird besonders darauf hingewiesen, dass die Zielzellfunktion "Superoxidanionenbildung" streng mit dem transformierten Phänotyp assoziiert und hochselektiv ist, da sie durch aktivierte Onkogene kontrolliert wird. Die Effektorfunktionen "Peroxidasefreisetzung" und "NO-Freisetzung" können jedoch sowohl von nichttransformierten als auch transformierten Zellen selbst ausgeführt werden. Daher läuft interzelluläres ROS-Signaling sowohl in der Interaktion zwischen transformierten und nichttransformierten Zellen (klassische interzelluläre Induktion der Apoptose), als auch autokrin zwischen transformierten Zellen ab.

Tumorzellen tragen an ihrer Außenseite ausreichend membranständige Katalase (CAT), die Wasserstoffperoxid zerstört und dadurch den HOCl-Weg (sowie den nicht eingezeichneten Nitrylchloridweg und die Metall-katalysierte Haber-Weiss-Reaktion) hemmt. Außerdem zerstört Katalase auch Peroxynitrit und interferiert dadurch wirkungsvoll mit dem NO/Peroxynitritweg. Eine zweite negative Interaktion der Katalase mit dem NO/Peroxynitritweg ist aus Gründen der Übersichtlichkeit nicht eingezeichnet: Compound I der Katalase kann NO zu NO₂ oxidieren und dadurch den NO/Peroxynitritweg hemmen.

### Figur 2

### Direkte Hemmung der protektiven Katalase führt zum selektiven Zelltod von Tumorzellen

Apoptoseinduktion durch ROS-Signaling kann in Tumorzellen durch direkte Hemmung der Katalase erfolgen. Die DE 103 58 077 offenbart Verfahren, die die Suche nach Katalasehemmstoffen ermöglichen.

### Figur 3

### Zerstörung der Katalase durch exogenen Singulettsauerstoff

ROS-Signaling kann durch direkte Applikation von Singulettsauerstoff-Generatoren (wie z. b. Photofrin) reaktiviert werden. Singulettsauerstoff reagiert mit Histidin des aktiven Zentrums der Katalase und führt dadurch zu deren Inaktivierung.

### Figur 4

### Modulation des NO-Stoffwechsels führt zu Singulettsauerstoff-vermittelter Katalaseinaktivierung

Durch Modulation des NO-Stoffwechsels kommt es nach mehreren Zwischenschritten zur Bildung von Singulettsauerstoff, Katalaseinaktivierung und Apoptose (PCT/EP2007/006964). Die Erhöhung der verfügbaren NO-Konzentration führt zu einer reversiblen Hemmung der Katalase. Dies gibt nun nicht umgesetztem Peroxynitrit und Wasserstoffperoxid die Möglichkeit, miteinander zu reagieren und dabei Singulettsauerstoff zu bilden. Dieser kann nun den FAS-Rezeptor ligandenunabhängig aktivieren, was unter anderem zu einer Steigerung der NOX-1-Aktivität führt. Daraus resultiert zwangsläufig eine Steigerung der Wasserstoffperoxidkonzentration, was wiederum die Entstehung von neuem Singulettsauerstoff und die Zerstörung von weiteren Katalasemolekülen ermöglicht.

### Figur 5

### Synergistische Effekte zwischen NOX-Stimulatoren und Modulatoren des NO-Stoffwechsels

Durch die Kombination von NOX-Stimulatoren und Modulatoren des NO-Stoffwechsels lassen sich synergistische Effekte erzielen (EPA 10.173500.9). Dies ist dadurch zu erklären, dass die parallele Steigerung der NO-Konzentration und der Superoxidanionenproduktion den FAS-Rezeptor-abhängigen Amplifikationsschritt unnötig macht und damit den Gesamtprozess vereinfacht und verstärkt.

### Figur 6

### Signalchemie nach Hemmung der Tumorzellkatalase mittels monoklonaler Antikörper gegen Katalase

Jeweils 12 500 MKN-45 Magenkarzinomzellen in 100 µl komplettem Medium blieben entweder unbehandelt (Kontrolle) oder erhielten 2mM Histidin (Singulettsauerstoff-Fänger), 50 µM Caspase-3-Inhibitor, 25 µM Caspase-8-Inhibitor, 25 µM Caspase-9-Inhibitor, 100 µM AEBSF (NADPH-Oxidase-Inhibitor), 100 U/ml MnSOD (Superoxidanionenfänger), 150 µM ABH (Peroxidasehemmstoff), 50 mM Taurin (HOCI-Fänger), 10 mM Mannitol (Hydroxylradikalfänger), 2.4 mM L-NAME (NOS-Inhibitor) oder 40 µM FeTPPS (Peroxynitritzerstörer). Anschließend wurden die angegebenen Konzentrationen monoklonaler Antikörper gegen humane Katalase zugegeben. Nach vier Stunden Inkubation bei 37 °C wurden jeweils in Doppelansätzen die Prozentsätze apoptotischer Zellen aufgrund der klassischen Apoptosemerkmale Kernkondensation, Kernfragmentation und Membrane Blebbing bestimmt. Dabei wurden pro Ansatz mindestens 200 Zellen bewertet. Kontrollansätze mit Kontroll-IgG statt monoklonalem Antikörper gegen Katalase wurden mitgeführt und zeigten keine Apoptoseinduktion (Daten nicht gezeigt).

Die Applikation von monoklonalem Antikörper gegen Katalase (nicht aber Kontrollantikörper) ergibt Apoptoseinduktion in Form einer Optimumskurve. Die Apoptoseinduktion ist unabhängig von Singulettsauerstoff (keine Hemmung durch Histidin), Caspase-8, aber abhängig von Caspase-9 und Caspase-3. Im gesamten Konzentrationsbereich ist die Reaktion von Superoxidanionen abhängig, da sowohl ein Hemmstoff der NADPH-Oxidase (AEBSF) als auch SOD zum Abbruch der Reaktion führen. Im niedrigen Konzentrationsbereich hemmen Inhibitoren des NO/Peroxynitritweges wie L-NAME (NOS-Inhibitor) und FeTPPS (Peroxynitritfänger). Im höheren Konzentrationsbereich hängt die Reaktion von HOCI ab (Hemmbarkeit durch den HOCI-Fänger Taurin und den Peroxidasehemmer ABH). Die Hemmbarkeit durch den Hydroxylradikalfänger Mannitol beweist die zentrale Rolle des Apoptoseinduktors Hydroxylradikal, der nach Zerfall von Peroxynitritsäure und nach Interaktion von HOCl mit Superoxidanionen entstehen kann.

### Figur 7

### Hemmung der Katalase durch Salicylsäure in MKN-45- oder Gumbuszellen (humane Lymphomalinie) ist unabhängig von Singulettsauerstoff und zeigt das gleiche Inhibitorprofil wie die Apoptoseinduktion durch Anti-CAT

Jeweils 12 500 MKN-45 Magenkarzinomzellen (A) oder 25 000 Gumbus Lymphomzellen (B) in 100 µl komplettem Medium blieben entweder unbehandelt (Kontrolle) oder erhielten 2mM Histidin (Singulettsauerstoff-Fänger), 50 mM Taurin (HOCI-Fänger) oder 2.4 mM L-NAME (NOS-Inhibitor). In Experiment A wurden außerdem 10 mM Mannitol (Hydroxylradikalfänger), 150 µM ABH (Peroxidasehemmstoff), 100 U/ml MnSOD (Superoxidanionenfänger) bzw. 25 µM FeTPPS (Peroxynitritzerstörer) eingesetzt.

Anschließend wurden die angegebenen Konzentrationen Salicylsäure zugegeben. Nach 5 Stunden (A) bzw. 4 Stunden Inkubation bei 37 °C wurden jeweils in Doppelansätzen die Prozentsätze apoptotischer Zellen aufgrund der klassischen Apoptosemerkmale Kernkondensation, Kernfragmentation und Membrane Blebbing bestimmt. Dabei wurden pro Ansatz mindestens 200 Zellen bewertet.

Das Ergebnis zeigt, dass Salicylsäure in beiden Zelllinien ROS-abhängige Apoptoseinduktion induziert, die unabhängig von Singulettsauerstoff ist. Im niedrigen Konzentrationsbereich der Salicylsäure läuft bevorzugt der NO/Peroxynitritsignalweg ab, bei höheren Konzentrationen dominiert der HOCI-Weg. Das Inhibitorprofil der durch Salicylsäure induzierten Apoptose in beiden Zelllinien gleicht dem Inhibitorprofil, das sich auch nach Einwirkung von Antikörpern gegen Katalase zeigt. Das Ablaufen der nachgewiesenen Signalchemie nach Salicylsäuregabe setzt zwingend voraus, dass es durch Salicylsäure zu einer Hemmung der protektiven Tumorzellkatalase gekommen sein muss, da sich sonst weder der HOCI- noch der NO/Peroxynitritweg hätten ausbilden können.

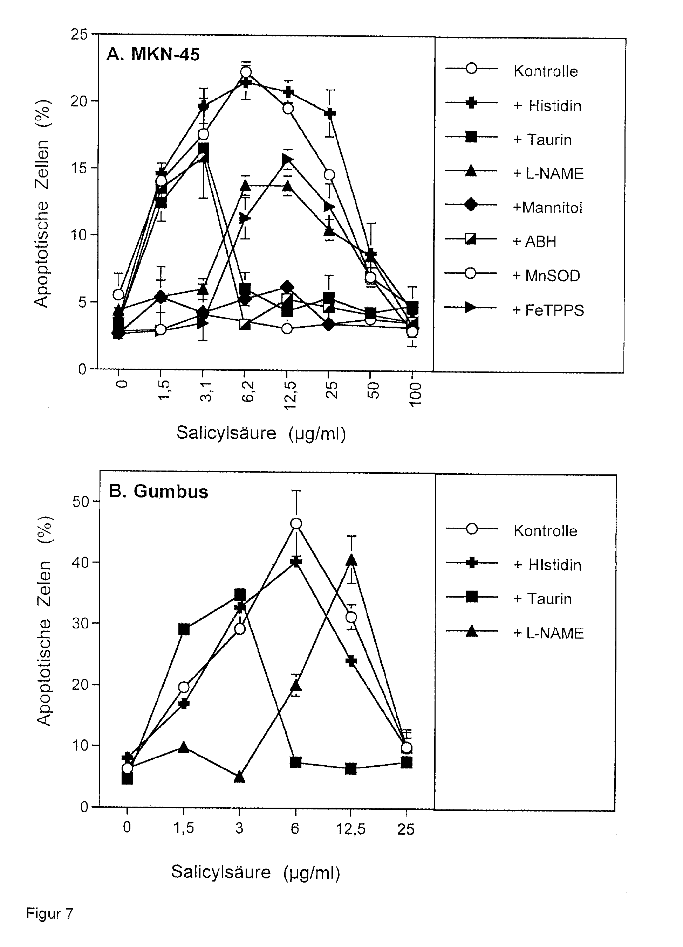

Die Figuren 6 und 7 zeigen die Wirkung von **direkten Katalasehemmstoffen** (nämlich monoklonalen Antikörpern, die gegen Katalase gerichtet sind, bzw. Salicylsäure) auf Tumorzellen. Diese Katalaseinhibitoren führen zur Reaktivierung der spezifischen interzellulären Signalwege durch Reaktive Sauerstoff- und Stickstoffspezies und nachfolgend zur Apoptose, wobei der Gesamtprozess unabhängig von Singulettsauerstoff ist.

### Figur 8

### Apoptoseinduktion durch Taxol beruht auf Singulettsauerstoff-abhängiger Zerstörung der Katalase

25 000 Gumbus Lymphomzellen in 100 µl Medium erhielten die angegebenen Inhibitoren entweder 10 Minuten vor Taxolzugabe (A) oder 1 Stunde danach (B). Doppelansätze wurden nach Taxolzugabe für 5 Stunden inkubiert, bevor die Prozentsätze apoptotischer Zellen ermittelt wurden. Inhibitoren: Singulettsauerstofffänger Histidin 2 mM; HOCI-Fänger Taurin 50 mM, NOS-Inhibitor L-NAME 2.4 mM, Peroxidasehemmstoff ABH 150 µM.

Die Apoptoseinduktion in Gumbuszellen durch Taxol wird durch einen schnellen Singulettsauerstoff-abhängigen Schritt bedingt, der innerhalb der ersten Stunde Katalase inaktiviert und dadurch nachfolgend ROS-Signaling erlaubt. Für die Singulettsauerstoffbildung sind Wasserstoffperoxid und Peroxynitrit notwendig. Das spätere Signaling erfolgt bei niedrigen Konzentrationen Taxol über den NO/Peroxynitritweg, bei höheren Konzentrationen über den HOCl-Weg.

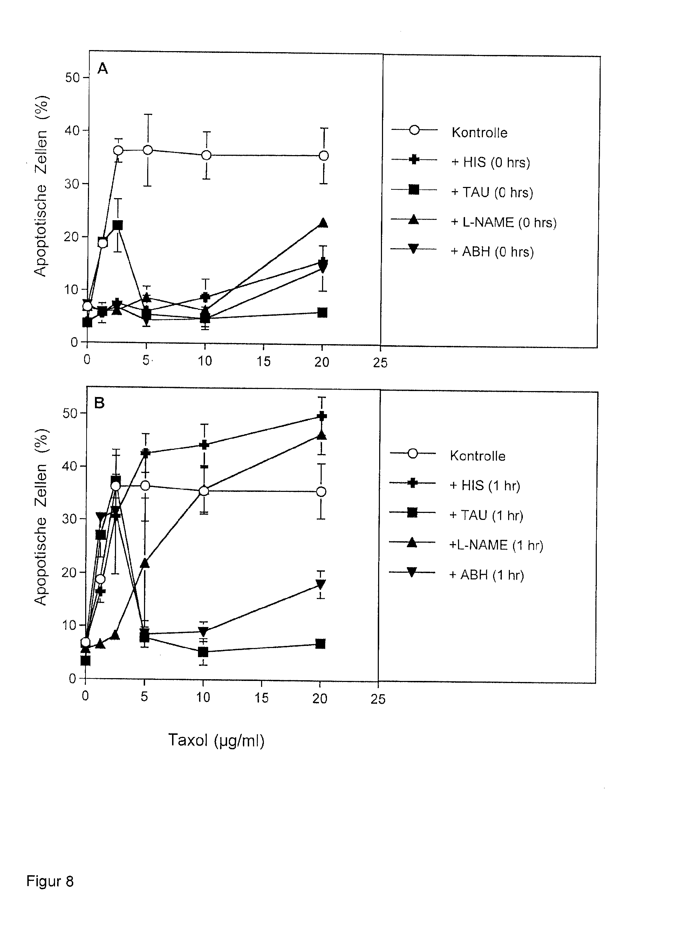

Figur 8 zeigt also, dass Taxol (das als NOD-Hemmstoff charakterisiert wurde) nur dann interzelluläres Signaling und Apoptose induzieren kann, wenn Singulettsauerstoffbildung möglich ist, wobei der Singulettsauerstoff-abhängige Prozess sehr früh innerhalb des Gesamtgeschehens ablaufen muss, um zu einer nachfolgenden Wirkung zu führen.

### Figur 9

### Direkter Nachweis der Katalasehemmung durch monoklonale Antikörper gegen Katalase

4000 MKN-45-Zellen pro 100 µl in 98-Lochplatten mit steigenden Konzentrationen von monoklonalen Antikörpern gegen Katalase "Anti-KAT" oder Anti-EGFR behandelt. Nach 15 Minuten erfolgte die Zugabe von entweder 200 µM Peroxynitrit ("PON"), 0.5 mM DEA-NONOate (schnell zerfallender NO-Donor) oder 2 mU/ml Glukoseoxidase (GOX), die stetig Wasserstoffperoxid generiert. Nach zwei weiteren Stunden bei 37 °C wurden die Prozentsätze apoptotischer Zellen jeweils in Doppelansätzen bestimmt.

Das Prinzip des Nachweises beruht darauf, dass Tumorzellen (hier MKN-45-Magenkarzinomzellen) durch ihre membranständige Katalase vor der Apoptoseauslösenden Wirkung von exogen zugegebenem Peroxynitrit geschützt sind. Zugabe von Katalasehemmern sensitiviert die Zellen für die Wirkung von exogenem Peroxynitrit. Da mit steigender Katalasehemmung vermehrt Wasserstoffperoxid freigesetzt wird, kommt es allmählich zu einem Verbrauch von Peroxynitrit durch Wasserstoffperoxid. Dies zeigt sich als Optimumskurve der Antikörperwirkung. Das Experiment belegt, dass der monoklonale Antikörper gegen Katalase die Tumorzellen durch Hemmung ihrer Katalase für exogenes Peroxynitrit, sowie für Peroxynitrit das durch die Interaktion von NO und Superoxidanionen entsteht, als auch für Wasserstoffperoxid sensitiviert.

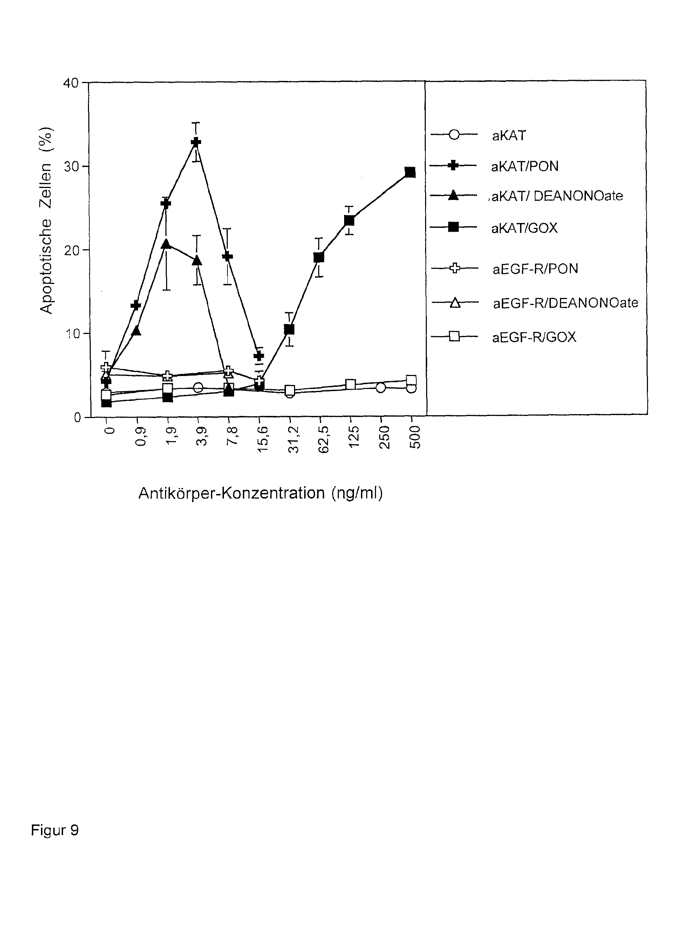

Figur 9 stellt den Nachweis der Katalaseinaktivierung bei Tumorzellen vor. Er beruht auf der Sensitivierung von Tumorzellen gegen exogen zugegebenem Peroxynitrit. Zur Spezifitätskontrolle wurden weitere Wirkstoffe, wie z. b. der NO-Donor DEA-NONOate und das H₂O₂-generierende Enzym Glucoseoxidase (GOX) untersucht. Die Verwendung von Peroxynitrit erlaubt die spezifische Messung der extrazellulären, membranständigen Katalase, da intrazelluläre Katalase (die nicht Tumorzell-spezifisch ist) extrazellulär zugegebenes Peroxynitrit nicht erreichen kann, bevor dieses zur Lipidperoxidation an der Zellmembran führt.

### Figur 10

### Anti-SOD führt zu einer Sensitivierung der Zellen durch Katalasehemmung

Das Experiment wurde analog dem in Figur 9 beschriebenen durchgeführt, außer dass statt Anti-KAT ein monoklonaler Antikörper gegen SOD eingesetzt wurde. Die Zellen wurden in zwei verschiedenen Dichten, nämlich 4000 bzw. 12 500 Zellen pro 100 µl getestet. Der Challenge zur Prüfung der Katalaseaktivität erfolgte durch 200 µM Peroxynitrit, oder 0.5 mM DEA NONOate oder 2 mU/ml GOX. Das Ergebnis (nach 2 Stunden Inkubationszeit) zeigt, dass auch Anti-SOD zur Hemmung der Katalaseaktivität führt. Parallel durchgeführte Untersuchungen zum Mechanismus dieser indirekten Wirkung zeigen, dass die nach SOD-Hemmung im Überschuss vorhandenen Superoxidanionen zu einer Hemmung der Katalase führen.

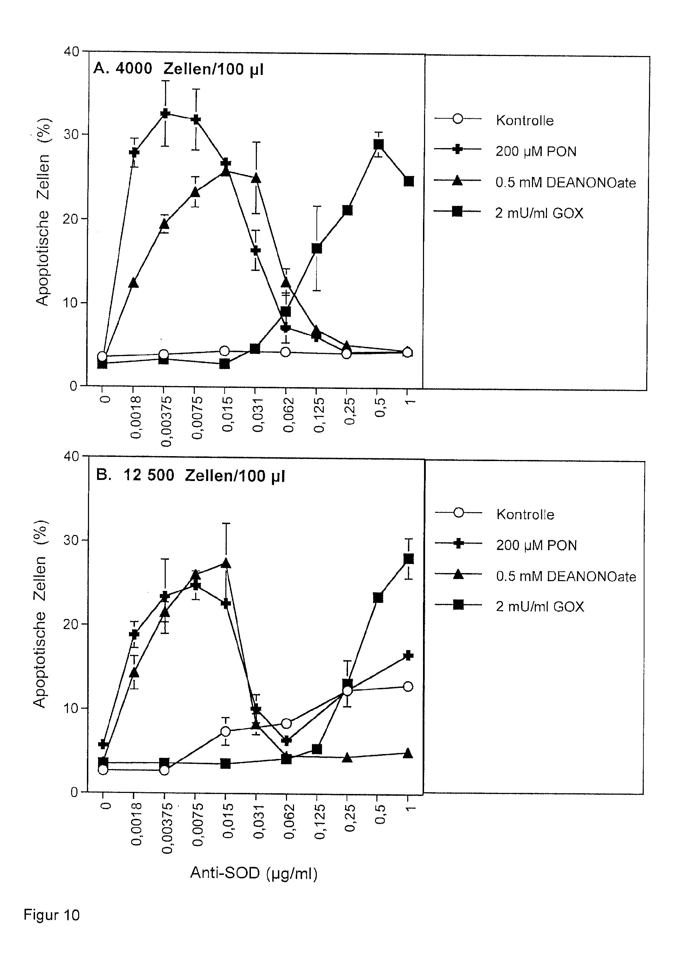

Figur 10 zeigt, dass die Hemmung der membranständigen SOD der Tumorzellen zwangsläufig auch zur Hemmung der membranständigen Katalase führt. Diese komplementäre Hemmung beruht darauf, dass die nach Hemmung der SOD in hoher Konzentration vorliegenden freien Superoxidanionen über einen Ein-Elektronenübergang die Katalase hemmen.

### Figur 11

### Salicylsäure hemmt die Tumorzellkatalase

12 500 MKN-Zellen pro 100 µl wurden mit den angegebenen Konzentrationen Salicylsäure für 15 Minuten vorbehandelt, in An- oder Abwesenheit von 100 µM AEBSF, welches wiederum 15 Minuten vor der Salicylsäure zu den Zellen gegeben worden war. Anschließend erfolgte die Zugabe von 200 µM Peroxynitrit oder keine Zugabe. Nach 1.5 Stunden wurden in den Doppelansätzen die Prozentsätze apoptotischer Zellen bestimmt. Das Experiment zeigt, dass Salicylsäure die Katalase der Tumorzellen hemmt. Die Hemmung erfolgt unabhängig von Superoxidanionensynthese. Wird die Superoxidanionensynthese jedoch gehemmt, bleibt der Konsum des Peroxynitrit durch Wasserstoffperoxid aus und die Optimumskurve wandelt sich in eine Plateaukurve um.

### Figur 12

### Hemmung der Tumorzellkatalase durch Methyldopa bzw. Ascorbinsäure

12 500 MKN-Zellen pro 100 µl wurden mit den angegebenen Konzentrationen Methyl-Dopa (A) bzw. Ascorbinsäure (B) für 15 Minuten vorbehandelt, in An- oder Abwesenheit von 100 µM AEBSF, welches wiederum 15 Minuten vorher zu den Zellen gegeben worden war. Anschließend erfolgte die Zugabe von 200 µM Peroxynitrit oder keine Zugabe. Nach 1 Stunde wurden in den Doppelansätzen die Prozentsätze apoptotischer Zellen bestimmt.

Das Experiment belegt, dass sowohl Methyl-Dopa als auch Ascorbinsäure Katalase direkt hemmen, ohne dass dazu die Anwesenheit von Superoxidanionen notwendig wäre. Dies lässt auch den Schluss zu, dass die Hemmung der Katalase unabhängig von Singulettsauerstoff erfolgt, da dessen Bildung die Verfügbarkeit von Superoxidanionen zwingend voraussetzt.

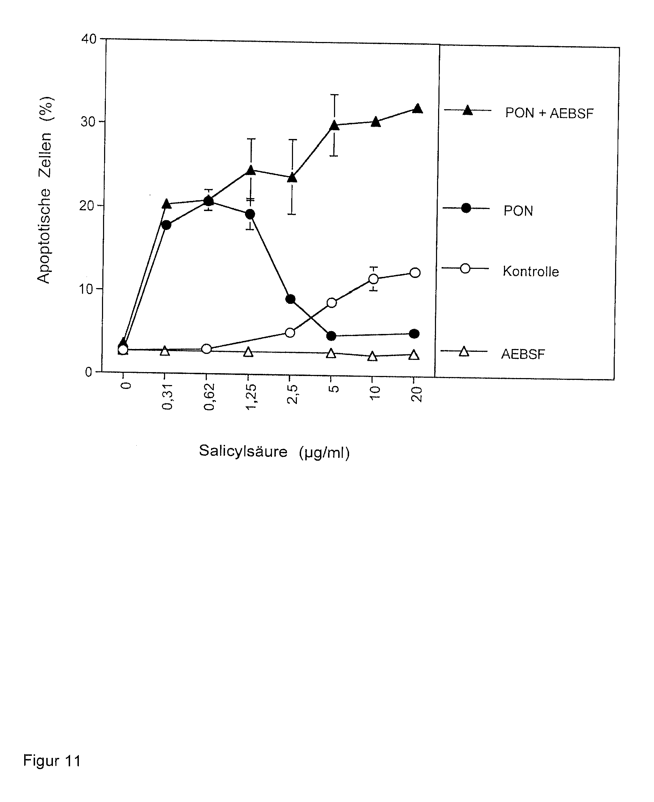

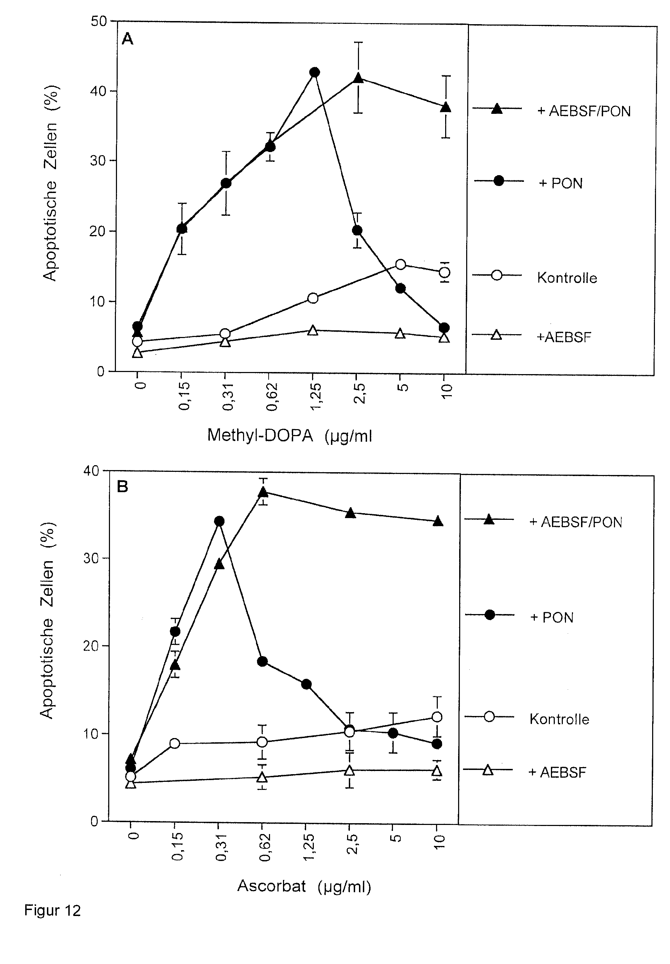

Die Figuren 11 und 12 belegen, dass Salicylsäure, Methyl-Dopa und Ascorbinsäure zu einer direkten Hemmung der Tumorzellkatalase führen. Die Sensitivierung der Tumorzellen findet auch dann statt, wenn durch Hemmung der NADPH-Oxidase mittels AEBSF keine Singulettsauerstoffbildung möglich ist.

### Figur 13

### Die Inaktivierung von Katalase durch Cyanidin benötigt Superoxidanionen

12 500 MKN-45-Zellen in 100 µl Medium erhielten 100 µM AEBSF oder keinen Zusatz. Nach 15 Minuten wurden die angegebenen Konzentrationen Cyanidin zugegeben und die Ansätze für 15 Minuten bei 37 °C inkubiert. Anschließend wurden 200 µM Peroxynitrit (PON) zugegeben oder die Ansätze nicht mit Peroxynitrit behandelt (Kontrolle). Nach 1 Stunde wurden in Doppelansätzen die Prozentsätze apoptotischer Zellen bestimmt.

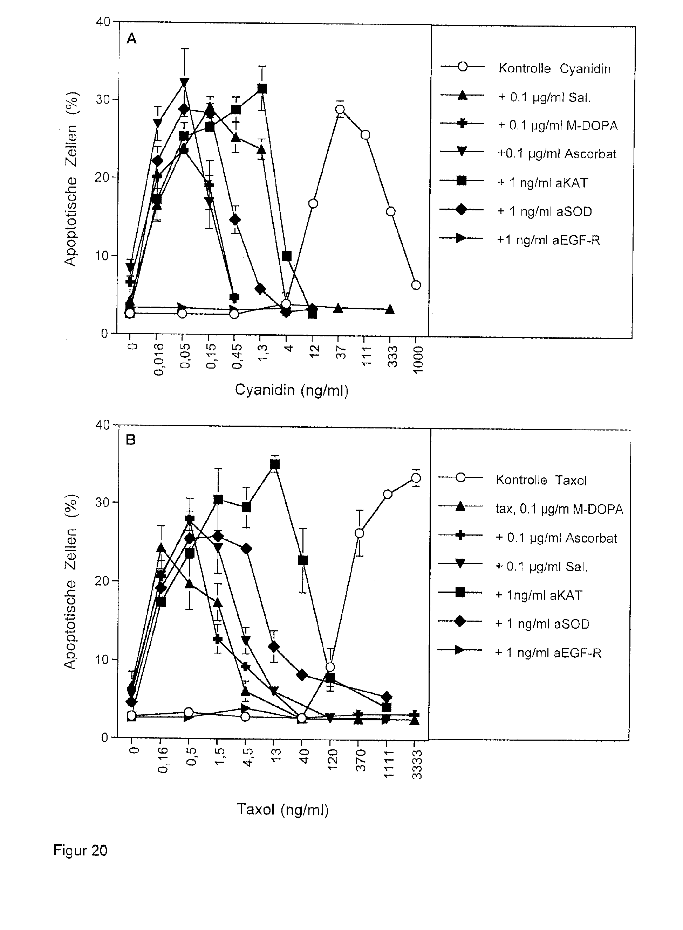

Das Ergebnis zeigt, dass die hemmende Wirkung von Cyanidin auf die Tumorzellkatalase von Superoxidanionen abhängig ist. Kontrolluntersuchungen zeigen, dass es sich hier um den komplexeren Mechanismus der Katalaseinaktivierung nach Bildung von Singulettsauerstoff handelt. Superoxidanionen sind dabei als Grundlage der Bildung von Wasserstoffperoxid und Peroxynitrit beteiligt. Die Interaktion dieser beiden Moleküle führt zur Bildung von Singulettsauerstoff. Dieser Sachverhalt wird in Figur 20 ausführlicher dargestellt.

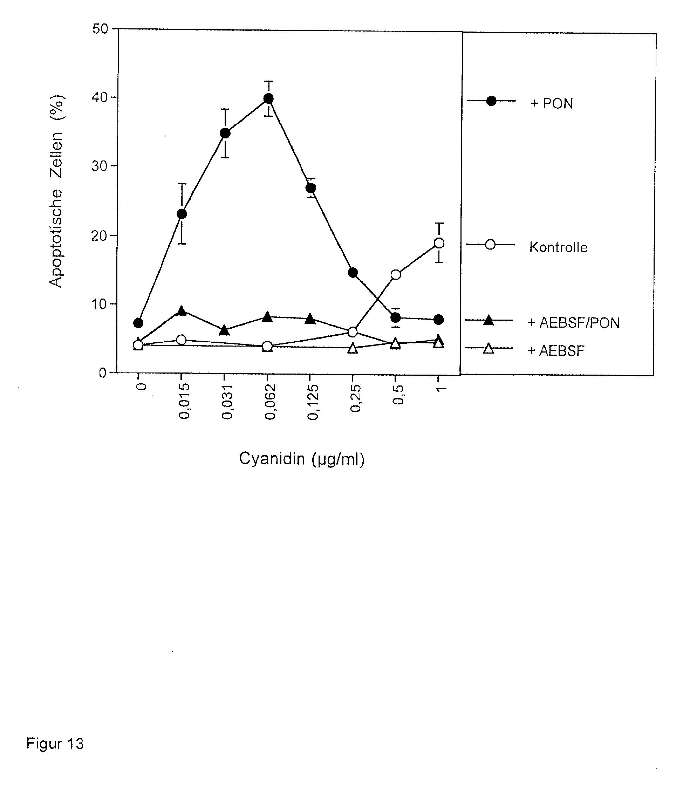

Figur 13 belegt, dass die sensitivierende Wirkung von Cyanidin (einem Hemmstoff der NOD) nur dann zum Tragen kommt, wenn Superoxidanionensynthese ablaufen kann. In Gegenwart des NOX-1-Inhibitors AEBSF zeigen die Tumorzellen keine Empfindlichkeit für Peroxynitrit.

### Figur 14

### Katalasehemmung durch Salicylsäure benötigt weder Singulettsauerstoff, noch Superoxidanionen, noch NO

12 500 MKN-45 Zellen erhielten keine Zugabe (Kontrolle), oder 2 mM Histidin, oder 100 µM AEBSF oder 2.4 mM L-NAME entweder unmittelbar vor Zugabe der angegebenen Konzentrationen Salicylsäure ("0 min") oder 20 Minuten nach Salicylgabe. 20 Minuten nach Salicylsäuregabe wurden 200 µM Peroxynitrit (PON) zu allen Ansätzen außer den Kontrollansätzen gegeben. Nach weiteren 2 Stunden wurden die Prozentsätze apoptotischer Zellen in Doppelansätzen bestimmt.

Das Experiment zeigt, dass die Zugabe des Singulettsauerstofffängers Histidin oder die Hemmung der NADPH-Oxidase durch AEBSF oder die Hemmung der NO-Synthase durch L-NAME zu einer Minderung der Hemmung der Katalase durch Salicylsäure führt. Dies belegt, dass die Hemmung der Katalase durch Salicylsäure nicht der Bildung von Singulettsauerstoff bedarf.

### Figur 15

### Inaktivierung von Katalase durch Cyanidin benötigt Superoxidanionen, NO und Singulettsauerstoff

12 500 MKN-45 Zellen erhielten keine Zugabe (Kontrolle), oder 2 mM Histidin, oder 100 µM AEBSF oder 2.4 mM L-NAME entweder unmittelbar vor Zugabe der angegebenen Konzentrationen Cyanidin ("0 min") oder 20 Minuten nach Cyanidingabe. 20 Minuten nach Cyanidingabe wurden 200 µM Peroxynitrit (PON) zu allen Ansätzen außer den Kontrollansätzen gegeben. Nach weiteren 2 Stunden wurden die Prozentsätze apoptotischer Zellen in Doppelansätzen bestimmt.

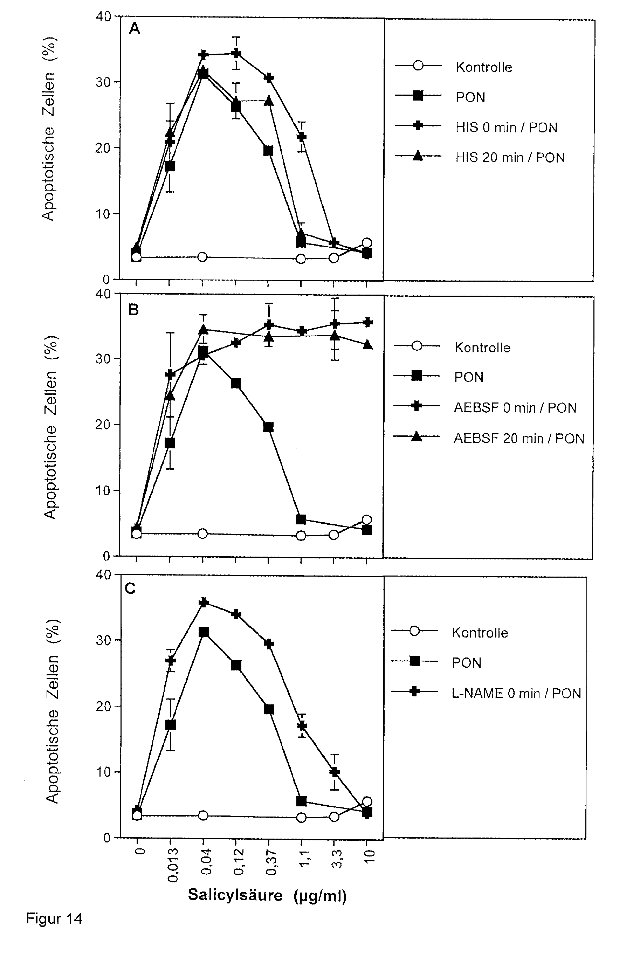

Im Gegensatz zu Salicylsäure (Figur 14) benötigt die Inaktivierung von Katalase durch Cyanidin Singulettsauerstoff, Superoxidanionen und NO. Die Wirkung dieser drei interagierenden Stoffe erfolgt früh, so dass nur eine Zugabe der Hemmstoffe zu Beginn der Applikation, nicht aber nach 20 Minuten die Inaktivierung der Katalase unterbinden kann.

Aufgrund der etablierten Chemie der Singulettsauerstoffbildung darf angenommen werden, dass Singulettsauerstoff durch die Reaktion von Peroxynitrit mit Wasserstoffperoxid gebildet wird. Die Hemmung der Superoxidanionsynthese hemmt somit sowohl die Peroxynitrit- als auch die Wasserstoffperoxid-Entstehung, während die Hemmung der NOS nur die Bildung des Peroxynitrits verhindert.

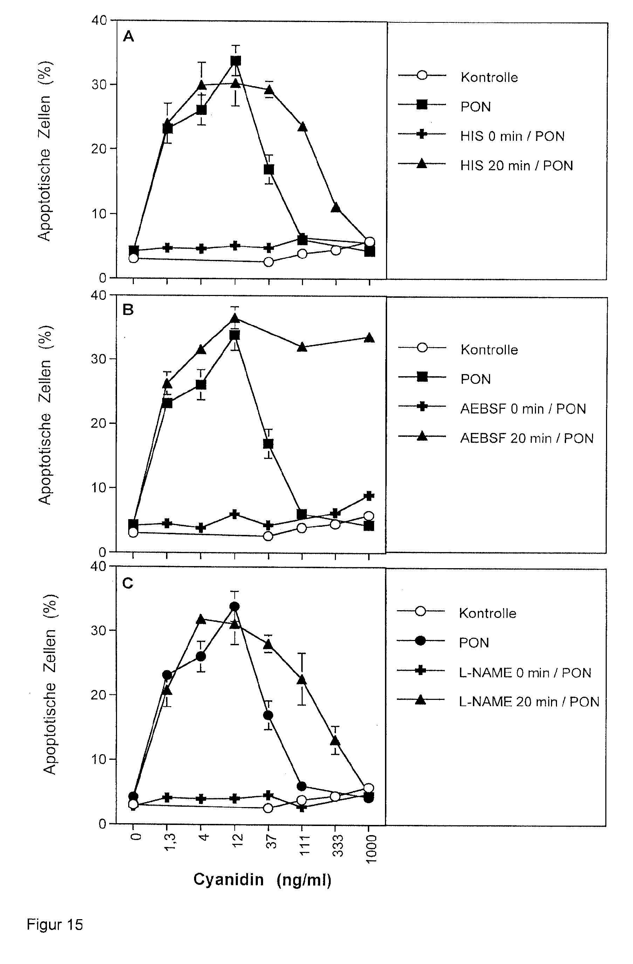

Die Differenzierung zwischen direkten Inhibitoren der Katalase und Wirkstoffen, die über Singulettsauerstoffbildung zur Inaktivierung der Katalase führen wird in den Figuren 14 und 15 weiter vertieft. Figur 14 belegt, dass die sensitivierende Wirkung von Salicylsäure davon *unabhängig* ist, ob Superoxidanionen gebildet werden können und ob sich der Singulettsauerstoff-Fänger Histidin im System befindet. Hingegen führt Cyanidin nur dann zu einer sensitivierenden Wirkung, wenn unmittelbar vor dem Peroxynitritechallenge Superoxidanionen-Synthese ablaufen darf und sich kein Histidin im System befindet, wie Figur 15 zeigt.

### Figur 16

### Synergistische Interaktion von Salicylsäure und Cyanidin bei der Apoptoseinduktion von MKN-45―Tumorzellen

12 500 MKN-45-Zellen in 100 µl (Doppelansätze) wurden mit den angegebenen Konzentrationen Salicylsäure behandelt oder blieben frei von Salicylsäure. Nach 10 Minuten erfolgte die Zugabe der angegebenen Konzentrationen Cyanidin. Die Auswertung nach 6 Stunden zeigt einen beachtlichen synergistischen Effekt bei kombinierter Gabe des Katalasehemmstoffs Salicylsäure und Cyanidin, das seinerseits die NOD hemmt und über einen komplexen Weg zur Singulettsauerstoffbildung und Katalaseinaktivierung führt.

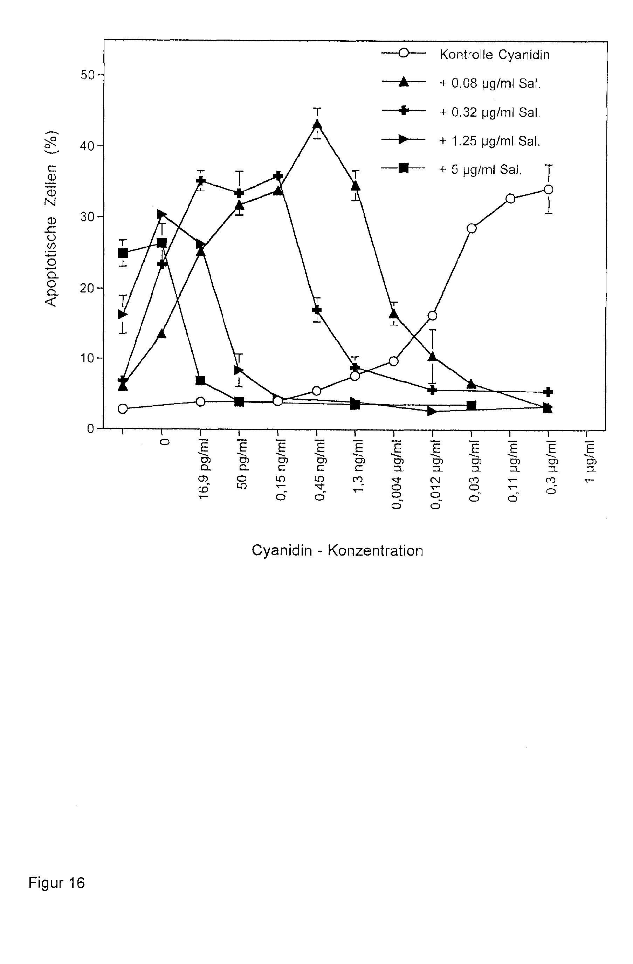

Figur 16 präsentiert die Konzentrationsverhältnisse bei der synergistischen Wirkung zwischen dem direkten Katalasehemmstoff Salicylsäure und dem Singulettsauerstoff-abhängigen NOD-Hemmstoff Cyanidin.

### Figur 17

### Synergismus zwischen Salicylsäure und Cyanidin bzw. Salicylsäure und Taxol bei der Apoptoseinduktion in Tumorzellen

12 500 MKN-45-Zellen in 100 µl (Doppelansätze) wurden entweder mit 0.1 µg/ml Salicylsäure behandelt oder blieben frei von Salicylsäure (Kontrolle). Nach 10 Minuten erfolgte die Zugabe der angegebenen Konzentrationen Cyanidin (A) oder Taxol (B). Nach 6.5 Stunden wurden die Prozentsätze apoptotischer Zellen in Doppelansätzen bestimmt.

Das Experiment zeigt, dass Salicylsäure sowohl mit dem Anthocyan Cyanidin, als auch mit dem etablierten Chemotherapeutikum Taxol einen bemerkenswerten synergistischen Effekt bewirkt.

### Figur 18

### Der synergistische Effekt zwischen Cyanidin und Salicylsäure ist Singulettsauerstoff-abhängig, aber unabhängig von Caspase-8

12 500 MKN-45-Zellen in 100 µl (Doppelansätze) erhielten entweder keinen Zusatz (Kontrolle), 2 mM des Singulettsauerstofffängers Histidin oder 25 µM Caspase-8-Inhibitor. Nach 15 Minuten erfolgte in dem unter B gezeigten Ansatz die Zugabe von 0.1 µg/ml Salicylsäure, während Ansatz A keine weiteren Zusätze erhielt. Nach weiteren 15 Minuten wurde Cyanidin in den angegebenen Konzentrationen zugegeben. Die Bestimmung des Prozentsatzes apoptotischer Zellen erfolgte nach 6.5 Stunden Inkubation bei 37 °C.

Das Experiment zeigt, dass die Wirkung von Cyanidin sowohl von Singulettsauerstoff, als auch von der Aktivität von Caspase-8 abhängig ist. Der ausgeprägte synergistische Effekt zwischen Cyanidin und Salicylsäure basiert ebenfalls auf der Wirkung von Singulettsauerstoff, ist jedoch unabhängig von Caspase-8.

### Figur 19

### Der synergistische Effekt zwischen Taxol und Salicylsäure ist Singulettsauerstoffabhängig, und im hohen Konzentrationsbereich von Taxol unabhängig von Caspase-8

12 500 MKN-45-Zellen in 100 µl (Doppelansätze) erhielten entweder keinen Zusatz (Kontrolle), 2 mM des Singulettsauerstofffängers Histidin oder 25 µM Caspase-8-Inhibitor. Nach 15 Minuten erfolgte in dem unter B gezeigten Ansatz die Zugabe von 0.1 µg/ml Salicylsäure, während Ansatz A keine weiteren Zusätze erhielt. Nach weiteren 15 Minuten wurde Taxol in den angegebenen Konzentrationen zugegeben. Die Bestimmung des Prozentsatzes apoptotischer Zellen erfolgte nach 6.5 Stunden Inkubation bei 37 °C.

Das Experiment zeigt, dass die Wirkung von Taxol bei allen Konzentration von Singulettsauerstoff abhängig ist. Eine notwendige Beteiligung von Caspase-8 ist jedoch nur bis 370 ng/ml Taxol erkennbar, bei höheren Taxolkonzentrationen wird Caspase-8 stetig weniger benötigt. Der synergistische Effekt zwischen Salicylsäure und Taxol ist komplett von Singulettsauerstoff abhängig, zeigt jedoch nur eine teilweise Beteiligung von Caspase-8.

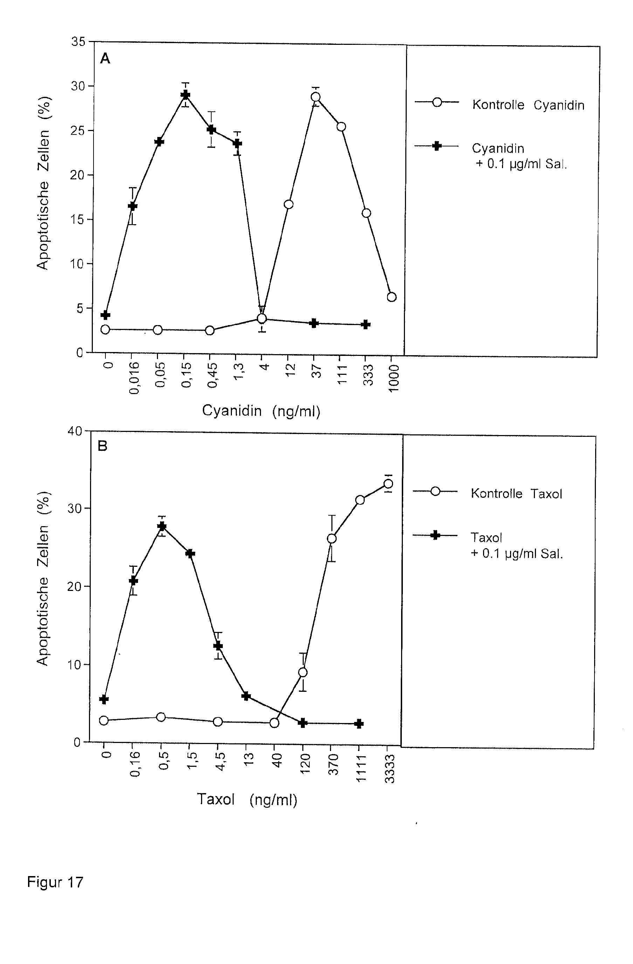

Die Figuren 17-19 vertiefen die Analyse der synergistischen Wirkung zwischen Salicylsäure auf der einen Seite und Cyanidin oder Taxol (beides NOD-Hemmstoffe) auf der anderen Seite. Dabei wird auch die Rolle des Singulettsauerstoffs und der Caspase-8 im Gesamtprozess beleuchtet.

### Figur 20

### Synergistische Wirkung verschiedener Katalasehemmer mit Cyanidin bzw. Taxol

12 500 MKN-45-Zellen in 100 µl (Doppelansätze) erhielten die angegebenen Konzentrationen der direkten Katalasehemmstoffe Salicylsäure, Methyl-dopa. Ascorbat, Anti-Katalase, Anti-SOD und den irrelevanten Antikörper Anti-EGF-Rezeptor. Kontrollansätze blieben frei von Hemmstoffen. Nach 15 Minuten erfolgte die Zugabe der angegebenen Konzentrationen Cyanidin (A) bzw. Taxol (B). Nach 6.5 Stunden Inkubation bei 37 °C wurden die Prozentsätze apoptotischer Zellen bestimmt.

Dieses Experiment zeigt, dass alle eingesetzten direkten Katalasehemmstoffe zu einer Synergiewirkung mit Cyanidin oder Taxol fähig sind.

Figur 20 zeigt die breite Anwendbarkeit des Konzeptes der hier vorgestellten Synergiewirkungen. Dabei werden fünf verschiedene direkte Inhibitoren auf ihre Synergiewirkung mit entweder Cyanidin oder Taxol untersucht.

### Figur 21

### Der synergistische Effekt zwischen Salicylsäure und Taxol führt zur Katalaseinaktivierung

12 500 MKN-45 Zellen wurden mit den angegebenen Konzentrationen Taxol, ohne und mit 6.25 ng/ml Salicylsäure für 15 Minuten behandelt, anschließend erfolgte die Zugabe von 200 µM Peroxynitrit. Der Prozentsatz apoptotischer Zellen wurde nach 1.5 Stunden gemessen. Es zeigt sich ein synergistischer Effekt auf die Katalaseinaktivierung.

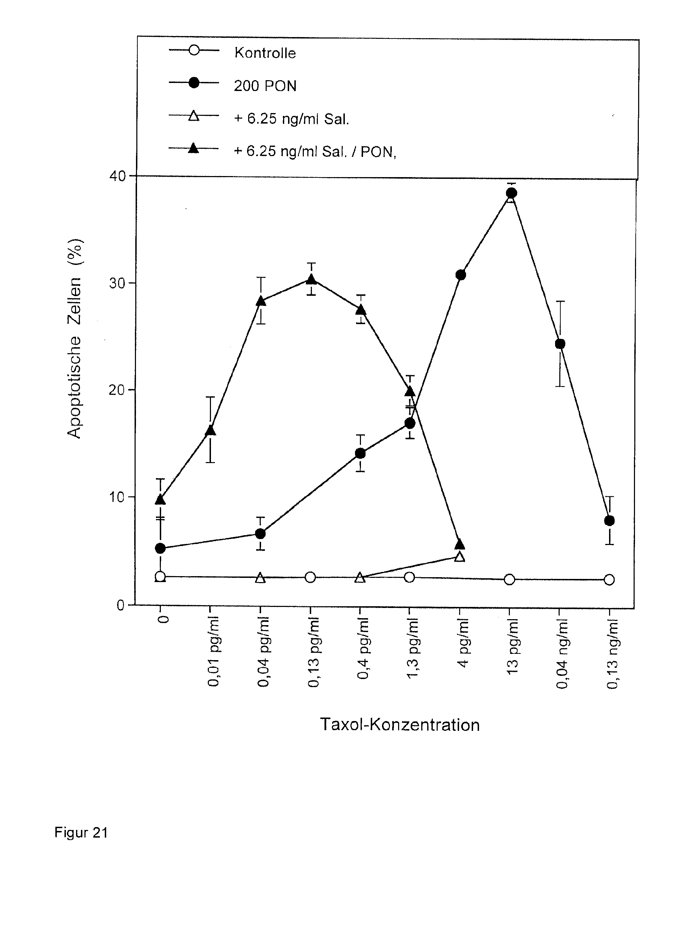

Figur 21 belegt, dass die Synergiewirkung zwischen Salicylsäure und Taxol auch dann nachweisbar ist, wenn die Inaktivierung der Tumorzellkatalase direkt gemessen wird.

### Figuren 22-25

### Der direkte Katalaseinhibitor Salicylsäure interagiert synergistisch mit einer Vielzahl von Modulatoren des NO-Stoffwechsels oder der NOX-1-Aktivität

12 500 MKN-45 Zellen in 100 µl Medium wurden mit steigenden Konzentrationen der angegebenen Wirkstoffe, in Abwesenheit oder Anwesenheit von 0.1 µg/ml Salicylsäure für 6 Stunden inkubiert und dann wurden die Prozentsätze der apoptotischen Zellen bestimmt (Doppelbestimmung).

Das Ergebnis zeigt, dass Salicylsäure synergistisch mit Arginin (Figur 22, oben), dem Arginasehemmstoff NOR-NOHA (Figur 22, unten), dem Stimulator der Superoxidanionenproduktion Resveratrol (Figur 23, oben), den NOD-Hemmstoffen Itraconazol, Artemisinin (Figur 23, unten), Diallyldisulfid ("DADS") (nicht aber dem inaktiven Diallylsulfid) ("DAS") (Figur 24, oben), Allylisothiocyanat ("AITC") (Figur 24, unten), Palmitinsäure (PS), sowie den Flavonoiden Quercetin (Figur 25, oben), Xanthohumol (XA) und Isoxanthohumol (IXA) (Figur 25, unten) reagiert.

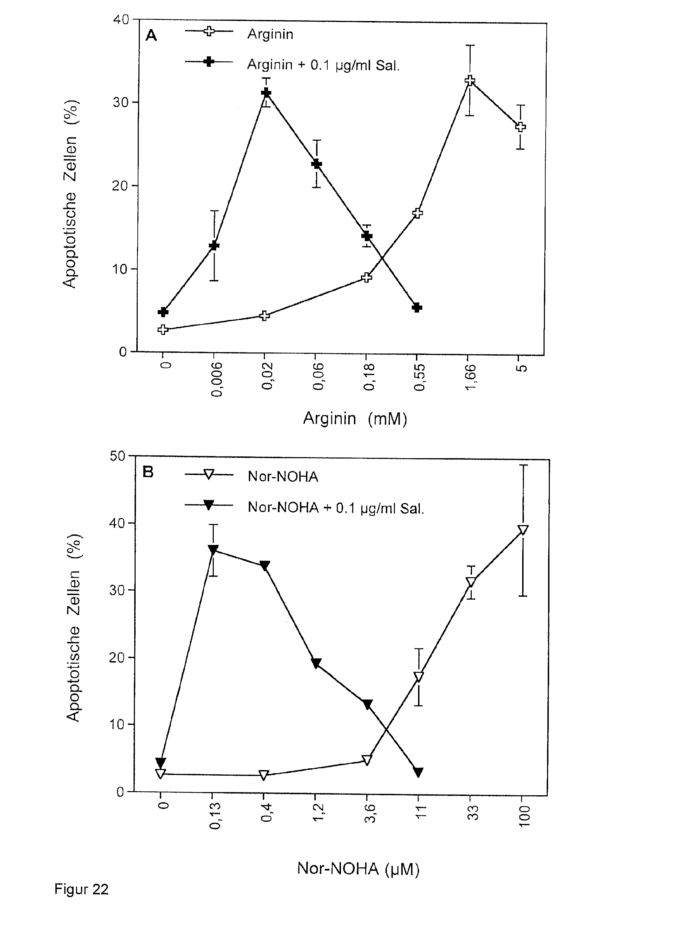

Die Figuren 22-25 führen weitere Beispiele für die breite Anwendbarkeit des hier vorgestellten neuen Konzeptes auf. Dabei wird eine für sich unwirksame Konzentration Salicylsäure mit einer Vielzahl von Modulatoren des NO-Stoffwechsels sowie mit dem NOX-1-Stimulator Resveratrol kombiniert. Dabei ergibt sich in jedem Fall eine sehr beeindruckende Synergiewirkung.

### Figur 26

### "Starterfunktion" der Katalasehemmung für die Katalaseinaktivierung durch Singulettsauerstoff

MKN-45 Zellen wurden mit jeweils 24 nM einer gegen humane Katalaseexpression gerichteten siRNA ("siCAT") oder einer irrelevanten Kontroll-SiRNA ("siCo") transfiziert und für 24 Stunden inkubiert. Die Details der Transfektion und der verwendeten siRNA sind in Heinzelmann und Bauer, 2010 beschrieben. Nach der Inkubation wurden die Zellen gewaschen. SiCo-transfizierte Zellen wurden als Reinkultur oder mit einem steigenden Anteil von siCAT-transfizierten Zellen angesetzt (insgesamt 12 500 Zellen / 100 µl Medium). Diese Ansätze erhielten entweder keine weiteren Zusätze (Control) oder Histidin (2mM), FeTPPS (25 µM) oder Taurin (50 mM). Die Zugabe dieser Inhibitoren erfolgte bei einigen der Ansätze direkt beim Zusammenmischen der unterschiedlich transfizierten Zellen (0) oder 20 Minuten später. Nach insgesamt 4.5 Stunden wurden die Prozentsätze apoptotischer Zellen in Doppelansätzen bestimmt.

Das Ergebnis zeigt, dass siCO-transfizierte Zellen nicht in Apoptose gehen, während siCAT-transfizierte Zellen knapp 40 % apoptotischer Zellen erreichen. Wurde ein kleiner Prozentsatz siCAT-transfizierter Zellen zu den siCo-transfizierten Zellen zugemischt, ergibt sich regelmäßig ein weitaus höherer Wert für die Apoptoseinduktion, als aus dem Mischungsverhältnis zu erwarten gewesen wäre.

Werden der Singulettsauerstofffänger Histidin oder der Peroxynitritfänger FeTPPS bereits beim Zusammenmischen der beiden Zellpopulationen zugegeben, ergibt sich jeweils ein Grad der Apoptoseinduktion, der dem Anteil der zugemischten siCAT-Zellen entspricht. Erfolgt die Zugabe der beiden Inhibitoren 20 Minuten später, ergibt sich fast das Bild des nicht inhibierten Kontrollansatzes.

Dieses Ergebnis zeigt also, dass ausgehend von wenigen Zellen mit fehlender Katalase eine quasiinfektiös sich ausbreitende Inaktivierung der Katalase der Nachbarzellen erreicht wird. Bei diesem "Bystandereffekt" spielen Peroxynitrit und der daraus hervorgehende Singulettsauerstoff eine essentielle Rolle.

### Figur 27

### Voraussetzungen für die interzelluläre Ausbreitung der Katalaseinaktivierung.

MKN-45 Zellen wurden mit folgenden siRNA-(Kombinationen) transfiziert:

Kontroll-siRNA (siCo), siRNA gegen Katalase (siCAT), siRNA gegen iNOS (siiNOS), siRNA gegen den FAS-Rezeptor (siFAS-R), einem Gemisch aus siCAT plus siiNOS [si(CAT+iNOS)] und einem Gemisch aus siCAT und siFAS-R [si(CAT + FAS-R)].

Nach 24 Stunden wurden die angegebenen Gemische aus den angegebenen Gruppen angesetzt (12 500 Zellen pro 100 µl Medium), inkubiert und nach 4.5 Stunden wurden die Prozentsätze apoptotischer Zellen bestimmt.

Das Experiment zeigt, dass die von den siCAT-transfizierten Zellen ausgehende Inaktivierung der Katalase der Nachbarzellen auch dann ablaufen kann, wenn die siCAT-transfizierten Zellen über keine iNOS oder keinen FAS-Rezeptor verfügen.

Hingegen hängt die Reaktionsfähigkeit der Zellen mit vorher intakter Katalase davon ab, dass sie über iNOS und FAS-Rezeptor verfügen. Dies weist insgesamt auf einen komplexen, in mehreren Stufen ablaufenden Prozess hin, bei dem ausgehend von wenigen Zellen mit fehlender Katalase in Nachbarzellen Katalaseinaktivierung unter Beteiligung des FAS-Systems und NO erfolgt.

### Figur 28

### Kinetische Analyse der interzellulären Ausbreitung der Katalaseinaktivierung

MKN-45-Zellen wurden mit siCO oder siCAT transfiziert (24 nM) und für 24 Stunden inkubiert. Danach wurden Reinkulturen der beiden Zellpopulationen oder ein Gemisch von 5 % siCAT-transfizierten mit 95 % siCo-transfizierten Zellen hergestellt. In allen Ansätzen wurde eine Zelldichte von 12 500 Zellen pro 100 µl eingestellt.

Die Ansätze blieben entweder ohne Inhibitoren oder erhielten 2 mM Histidin, 2.4 mM L-NAME oder 50 mM Taurin von Anfang an (0) oder nach fünf Stunden (5). Die Bestimmung der Prozentsätze apoptotischer Zellen erfolgte zu den angegebenen Zeitpunkten.

Das Ergebnis zeigt, dass siCAT-transfizierte Zellen sehr schnell in Apoptose gehen, wobei dann bereits nach 2 Stunden ein Abbruch der Reaktion erfolgt. In Gegenwart von Histidin erfolgt der Anstieg der Apoptosekurve geringfügig verzögert, der Abbruch erfolgt aber später und auf einem höheren Apoptoseniveau. Histidin verlangsamt also geringfügig die Apoptosekinetik, verhindert aber zunächst auch, dass die Reaktion aus dem Optimum läuft. SiCo-transfizierte Zellen zeigen keine Apoptoseinduktion im Beobachtungszeitraum. Wurden zu den siCo-transfizierten Zellen 5 % siCAT-transfizierte Zellen gemischt, ergibt sich nach einer dreistündigen Latenzzeit ein steiler Anstieg der Apoptosekinetik. Dieser Anstieg wird komplett verhindert, wenn vom Zeitpunkt 0 an Histidin im System war. Zugabe von Histidin zum Zeitpunkt 5 Stunden hat hingegen keinerlei Wirkung auf die Apoptoseinduktion. Das gleiche Muster ergibt sich für die Wirkung des NOS-Inhibitors L-NAME. Die Zugabe von Taurin zum Zeitpunkt 0 Stunden führt zu einer fast vollständigen Hemmung der Apoptose, Zugabe zum Zeitpunkt 5 Stunden erreicht jedoch, im Gegensatz zu den beiden anderen Inhibitoren, einen sofortigen Abbruch der Apoptoseinduktion. Dieses komplexe Geschehen lässt sich folgendermaßen erklären: Innerhalb der Latenzphase kommt es ausgehend von den siCAT-Zellen zu einer Inaktivierung der Katalase bei siCo-Zellen, wobei Singulettsauerstoff und NO eine zentrale Rolle spielen. Die Inaktivierung der Katalase in der Gesamtpopulation der Zellen erlaubt nun das Ablaufen des HOCl-Weges, der zu jedem Zeitpunkt durch Taurin unterbrochen werden kann.

### Figur 29

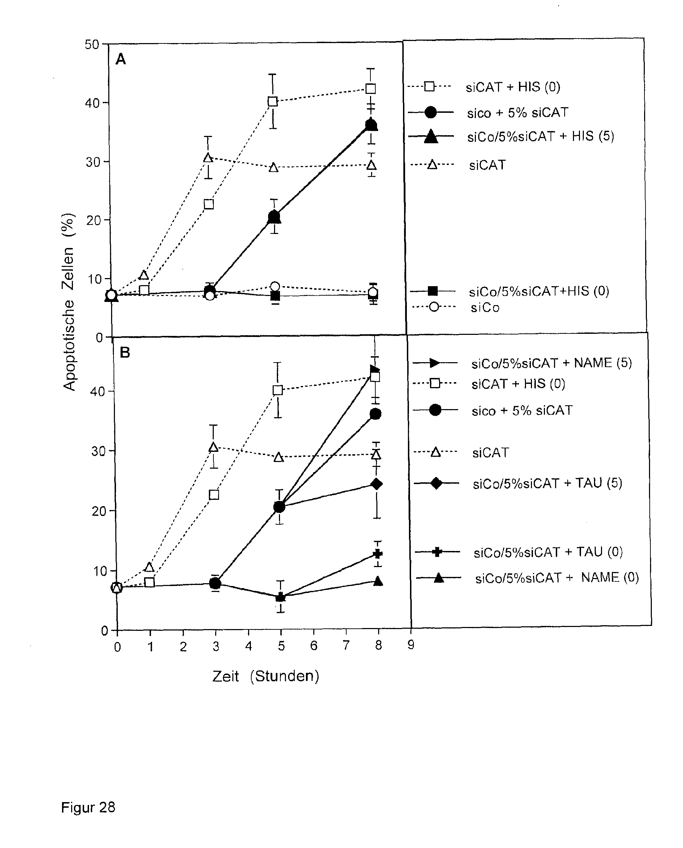

### Kontrolluntersuchungen zu dem in Figur 28 gezeigten Experiment

Der Versuchsaufbau entspricht dem in Figur 28 beschriebenen Experiment. Es handelt sich um die selben Zellpopulationen (siCo und siCAT).

Die Daten zeigen, dass die Supplementation der siCAT-Zellpopulation mit exogener Katalase ("CAT", 30 U/ml) den Effekt des siRNA-abhängigen Katalaseknockdowns wieder aufhebt und damit dessen Spezifität beweist. Bei der Apoptoseinduktion in siCAT-transfizierten Zellen, mit oder ohne Histidinzugabe, handelt es sich vorwiegend um den HOCl-Weg (Hemmbarkeit durch Taurin) und nur in geringfügigem Maß um eine Beteiligung von NO.

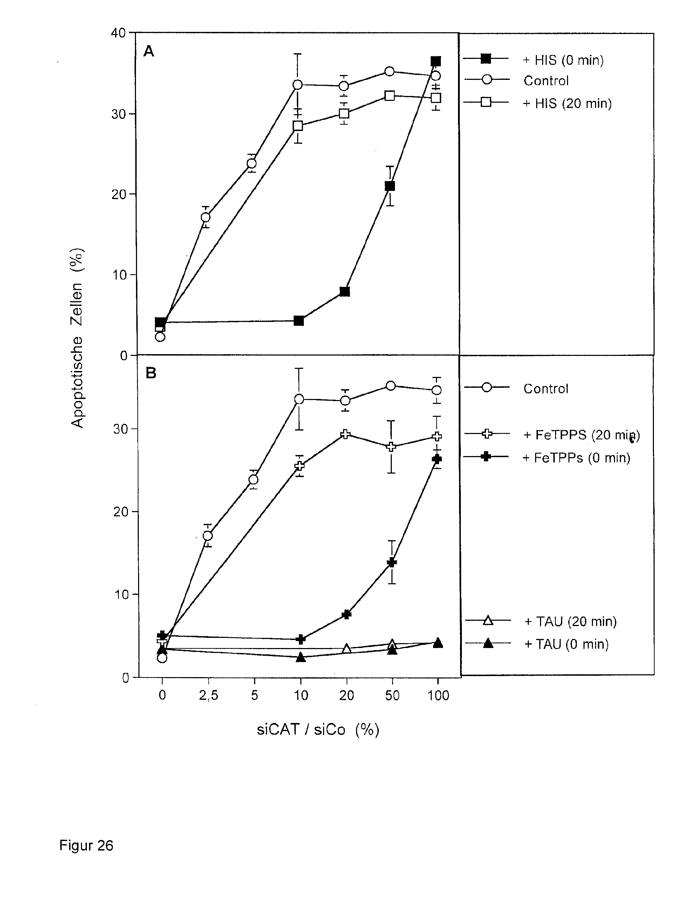

Die Figuren 26-29 stellen Modellexperimente vor, die die "quasiinfektiöse" Ausbreitung der Katalaseinaktivierung ausgehend von Tumorzellen mit fehlender Katalase (siRNA-Knockdown) innerhalb der Zellpopulation mit ursprünglich intakter, protektiver Katalase. Dieser Prozess wird durch Singulettsauerstoff vermittelt und ist für das Verständnis der zunächst unerwarteten synergistischen Wirkung zwischen direkten Katalasehemmstoffen auf den durch NO- oder Superoxidanionen-modulierenden Wirkstoffen, die Katalase mittels Singulettsauerstoffbildung zerstören, essentiell.

### Beispiel 1

### 1.1 Charakterisierung der unterschiedlichen Wirkungen auf Katalase

Figur 6 fasst die wesentlichen Charakteristika der Apoptoseinduktion in der Magenkarzinomzelllinie MKN-45 nach Gabe eines direkt die Katalase hemmenden Wirkstoffs, nämlich des monoklonalen Antikörpers gegen Katalase, zusammen. Es erfolgt Apoptose in Abhängigkeit von der Antikörperkonzentration bis zu einem Optimum, anschließend ergibt sich ein supraoptimaler Abstieg der Kurve. Die Apoptoseinduktion nach Antikörper-vermittelter Katalasehemmung erfolgt *unabhängig von Singulettsauerstoff*, da keine Hemmung durch Histidin zu beobachten ist. Über den gesamten Konzentrationsbereich des Antikörpers ist die Apoptoseinduktion von extrazellulären Superoxidanionen abhängig, da sie durch AEBSF, einem NADPH-Oxidasehemmstoff und durch SOD vollständig blockiert wird. Die intrazellulären Abläufe der Apoptose werden durch Caspase-9 und Caspase-3 vermittelt, was den Rückschluss auf den mitochondrialen Weg der Apoptose zulässt. Todesrezeptoren sind erwartungsgemäß nicht an der Apoptoseinduktion beteiligt, da Caspase-8-inhibitor keine Hemmwirkung zeigt. Figur 6B belegt, dass im niedrigen Konzentrationsbereich des Antikörpers bevorzugt der NO/Peroxynitritweg abläuft, da eine Hemmung der Apoptose erfolgt, wenn die NO-Synthase durch L-NAME blockiert oder entstehendes Peroxynitrit durch FeTPPS (einem katalytisch wirkenden Peroxynitritzersetzer, "peroxynitrite decomposition catalyst") zerstört wird. Mit steigender Antikörperkonzentration wird der NO/Peroxynitritweg durch den HOCl-Weg abgelöst, wie aus der Hemmbarkeit durch ABH, einen Peroxidasehemmstoff und durch Taurin, einem HOCI-Fänger, zu erkennen ist. Die Hemmbarkeit durch den Hydroxylradikalfänger Mannitol im gesamten Konzentrationsbereich des Antikörpers erklärt sich daraus, dass Hydroxylradikale bei beiden Signalwegen das eigentliche Apoptose-auslösende Radikal darstellen. Beim NO/Peroxynitritweg entstehen diese durch homolytischen Zerfall der Peroxynitritsäure, beim HOCl-Weg nach Interaktion zwischen HOCI und Superoxidanionen.

Figur 7 demonstriert, dass Salicylsäure in der Magenkarzinomzelllinie MKN-45 und der Lymphomzelllinie Gumbus in ähnlicher Weise und aufgrund der gleichen Signalwege Apoptose auslöst, wie dies in der vorangegangenen Figur 6 für gegen Katalase gerichtete Antikörper demonstriert und vor kurzem auch für den Katalasehemmstoff 3-Aminotriazol publiziert wurde (Heinzelmann and Bauer, 2010). Die fehlende Hemmbarkeit durch Histidin weist auch hier auf die direkte Hemmung der Katalase durch Salicylsäure, unabhängig von einer Singulettsauerstoffwirkung hin. Salicylsäure wurde durch Screening mithilfe des in DE 103 58 077 A1 2005.07.28 beschriebenen Verfahrens als Hemmstoff für die Katalase humaner Tumorzellen charakterisiert. Durch das gleiche Testsystem wurden auch Ascorbinsäure und M-Dopa als direkte Katalasehemmstoffe identifiziert.

Der direkten Hemmung der Tumorzellkatalase steht die indirekte, durch Modulation des NO-Stoffwechsels und nachfolgender Singulettsauerstoffbildung bedingte Inaktivierung der Katalase entgegen. Sie wird in der Figur 8 exemplarisch für Taxol dargestellt. Analoge Daten liegen für eine Reihe weiterer Naturstoffe, darunter Flavonoide, Anthocyane, Diallyldisulfid, Artemisisin, Palmitinsäure, Isothiocyanate wie z.B. Methylisothiocyanat, Allylisothiocyanat und Sulforaphan, sowie verschiedene Azole wie z. b. Itraconazol, Econazol, Fluconazol, Mikonazol, Ketokonazol vor. Figur 8 zeigt, dass die Apoptose-auslösende Wirkung von Taxol gehemmt wird, wenn bei Zugabe von Taxol Singulettsauerstoff durch Histidin abgefangen oder die NO-Synthase blockiert wird. Eine Stunde später verändert sich das Inhibitionsprofil und die Reaktion weist wieder die Aufteilung in den NO/Peroxynitritweg und nachfolgend den HOCI-Weg auf. Weitere Modelluntersuchungen führen insgesamt zu dem Bild, dass in einem ersten Schritt Singulettsauerstoff gebildet wird, der die Katalase inaktiviert, wodurch dann das klassische Signaling über die bekannten Signalwege erlaubt wird. NO und das daraus gebildete Peroxynitrit haben eine zweifache Funktion in diesem System. Zu Beginn der Taxolbehandlung werden NO und Peroxynitrit als Komponenten zur Singulettsauerstoffbildung im gesamten Konzentrationsbereichs des Taxols benötigt. Nach der sehr schnell erfolgenden Inaktivierung der Tumorzellkatalase werden dann NO und Peroxynitrit für das eigentliche interzelluläre Apoptosesignaling nur im niedrigen Konzentrationsbereich des Taxol benötigt, während bei höheren Konzentrationen Taxol der HOCI-Weg wirksam wird.

In den in Figuren 9-15 dargestellten Experimenten wird die Wirkung verschiedener Stoffgruppen auf die Katalase direkt untersucht. Dazu werden Tumorzellen mit steigender Konzentration der zu testenden Substanz behandelt und anschließend mit einem Überschuss an exogenem Peroxynitrit versehen. Bei intakter Katalase erfolgt keine Apoptoseinduktion in den Tumorzellen, bei gehemmter oder inaktivierter Katalase jedoch sehr schnell Apoptoseinduktion durch das nicht mehr zerstörte Peroxynitrit. Aufgrund der schnellen Reaktion nach exogener Peroxynitritgabe wird bei dieser Vorgehensweise die autokrine Apoptoseinduktion der Zellen weitgehend ausgeblendet und es erfolgt eine wertvolle Fokussierung auf die Katalasewirkung.

Figur 9 zeigt, dass Antikörper gegen Katalase, nicht aber irrelevanter Antikörper (hier gegen EGF-Rezeptor) die Zellen für die Peroxynitritwirkung sensitiviert. Die Reaktion zeigt das Bild einer Optimumskurve, was durch die negative Wirkung von überschüssigem Wasserstoffperoxid auf Peroxynitrit erklärt werden kann (Heinzelmann and Bauer, 2010). Das gleiche Bild ergibt sich, wenn statt exogenem Peroxynitrit ein NO-Donor (DEA-NONOate) appliziert wird. Das von ihm sehr schnell freigesetzte NO reagiert mit den extrazellulär durch NOX-1 generierten Superoxidanionen zu Peroxynitrit. Bei höheren Antikörperkonzentrationen werden die Zellen schließlich für die direkte Apoptoseinduktion durch einen exogenen Wasserstoffperoxid-Generator (Glucoseoxidase) sensitiviert. Diese Befunde bestätigen also die Katalase-hemmende Wirkung des monoklonalen Antikörpers gegen Katalase.

Figur 10 zeigt, dass Antikörper gegen SOD den gleichen Effekt auf die Katalase auslösen kann wie zuvor in Figur 9 für Anti-CAT gezeigt. Dieser unerwartete Befund wird dadurch erklärt, dass die membranständige Katalase der Tumorzellen durch eine membranständige SOD bei der Hemmung der interzellulären Signalwege unterstützt wird (EPA 11.170076.1). Nach Hemmung der SOD führen die jetzt lokal in sehr hoher Konzentration vorliegende Superoxidanionen jedoch zu einer Hemmung der benachbarten Katalase. Dies führt nun zur eigentlichen Sensitivierung der Zellen für die Wirkung von Peroxynitrit.

Figur 11 belegt, dass Salicylsäure ebenfalls zu einer Hemmung der Katalase führt, die sich in einer Sensitivierung gegenüber exogenem Peroxynitrit äußert. Bei gleichzeitiger Hemmung der NADPH-Oxidase durch AEBSF fällt die Konsumreaktion zwischen Peroxynitrit und Wasserstoffperoxid weg und die Optimumskurve wird in eine Plateaukurve umgewandelt. Der gleiche Sachverhalt, also direkte Katalasehemmung, trifft für Methyldopa und Ascorbinsäure zu, wie Figur 12 belegt wird.

Auch das Einwirken des Anthocyans Cyanidinchlorid führt zu einer Sensitivierung für Peroxynitrit, wie Figur 13 zeigt. Bei Hemmung der Superoxidanionenproduktion durch AEBSF bleibt die Sensitivierung durch Cyanidin jedoch erfolglos. Hier ist also eine klare Trennung zum Wirkmechanismus der vorher diskutierten Gruppe (Salicylsäure, Methyldopa, Ascorbinsäure) möglich. Dies ist dadurch erklärt, dass hier keine direkte Katalasehemmung erreicht wird, sondern ein komplexerer, ROS-abhängiger Katalaseinaktivierungsschritt. Es handelt sich dabei um den Singulettsauerstoff-abhängigen Inaktivierungsschritt der von zahlreichen Modulatoren des NO-Stoffwechsels eingeleitet wird.

Der biochemische Unterschied zwischen direkter Katalasehemmung (unabhängig von ROS-Interaktionen und Singulettsauerstoffbildung) und indirekter, auf Katalasezerstörung begründeter Sensitivierung unter Beteiligung von ROS-Interaktionen und Singulettsauerstoff wird in Figuren 14 und 15 vorgestellt. Während die hemmende Wirkung von Salicylsäure auf Tumorzellkatalase unabhängig von Superoxidanionen, NO und Singulettsauerstoff erfolgt (Figur 14), zeigt sich eine starke Abhängigkeit der Cyanidinwirkung von Superoxidanionen, NO und Singulettsauerstoff (Figur 15).

### 1.2 Synergistische Wirkung von Katalasehemmstoffen und Modulatoren des NO-Stoffwechsels oder der extrazellulären Superoxidanionenproduktion

Die synergistische Wirkung zwischen einem direkten Katalasehemmstoff (hier exemplarisch Salicylsäure) und einem NO-Modulator, der zu einer Singulettsauerstoff-abhängigen Katalasezerstörung führt (hier exemplarisch Cyanidin) wird in Figur 16 systematisch beleuchtet. Cyanidin führt, alleine appliziert, zu konzentrationsabhängiger Apoptoseinduktion, die etwa bei 0.02 µg/ml ihren halbmaximalen Wert und bei 0.3 µg/ml ihr Optimum erreicht. Bei alleiniger Gabe zeigt Salicylsäure bis 0.32 µg/ml fast keine Wirkung und dann ansteigende Wirkung bei höheren Konzentrationen. Die Kombination dieser beiden Stoffe führt zu einem außerordentlich deutlichen synergistischen Effekt. So erlaubt z.B. die Kombination von 0.15 ng/ml Cyanidin mit 0.08 µg/ml Salicylsäure maximale Apoptoseinduktion, während die Anwendung der beiden Stoffe einzeln und in der gleichen Konzentration wirkungslos blieb.

Die Wiederholbarkeit und eindrucksvolle Stärke dieses Synergismus wird in Figur 17 für das Wechselspiel zwischen einer niedrigen und daher für sich allein wirkungslosen Konzentration Salicylsäure auf der einen Seite und Cyanidin oder Taxol auf der anderen Seite demonstriert.

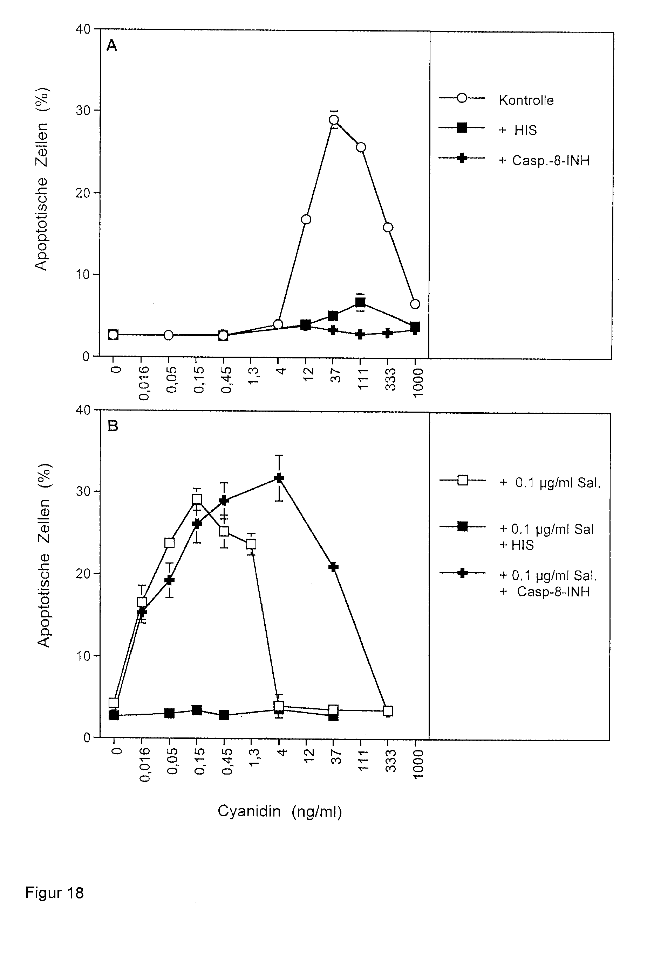

Figur 18 belegt, dass die Wirkung von Cyanidin alleine sowohl von der Aktivität von Todesrezeptoren (wie FAS) und der dadurch aktivierten Caspase-8, als auch vom Entstehen von Singulettsauerstoff abhängig ist. Bei der synergistischen Wirkung zwischen Salicylsäure und Cyanidin handelt es zwar ebenfalls um einen Singulettsauerstoff-abhängigen Prozess, was die Hemmbarkeit durch Histidin belegt, doch wird nun kein Caspase-8-abhängige Verstärkung der Signalwege benötigt.

Dieses Experiment zeigt, dass die für sich allein unwirksame Konzentration von 0,1 µg/ml Salicylsäure den durch Cyanidin gesteuerten, Singulettsauerstoff-abhängigen Prozess wesentlich effektiver macht und dadurch die Konzentrationsabhängigkeit für Cyanidin weit nach links verschiebt. Außerdem kann auf den Caspase-8-abhängigen Amplifikationsschritt verzichtet werden, wenn Cyanidin und Salicylsäure synergistisch miteinander kooperieren.

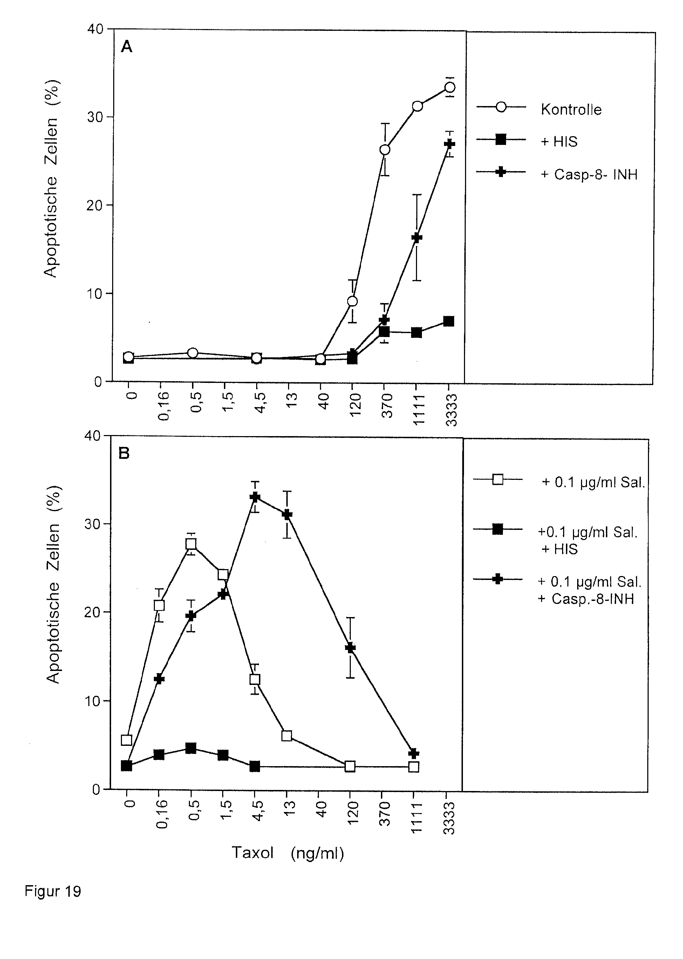

Das Bild wird noch differenzierter, wenn die Interaktion von Taxol mit Salicylsäure und die Taxolwirkung alleine beleuchtet werden (Figur 19). Die Taxolwirkung alleine beruht auf Singulettsauerstoff und der Wirkung von Caspase-8 im niedrigen Konzentrationsbereich, nicht aber bei höheren Konzentrationen. Beim synergistischen Effekt ergibt sich das gleiche Bild wie vorher für die Interaktion Salicylsäure und Cyanidin gezeigt. Diese Daten zeigen, dass der unter bestimmten Bedingungen notwendige Verstärkungseffekt durch Caspase-8 verzichtbar wird, wenn ein subtoxische Konzentration eines Katalasehemmstoffes mit einem Modulator der NO-Konzentration zusammenwirkt.

Die sehr breite Anwendbarkeit und Allgemeingültigkeit des hier beschriebenen neuen Verfahrens wird in Figur 20 dargestellt. Sowohl Cyanidin als auch Taxol zeigen hier einen beachtlichen synergistischen Effekt wenn sie mit subtoxischen Konzentrationen mehrerer Wirkstoffe kombiniert werden, für die oben eine direkte hemmende Wirkung auf Katalase nachgewiesen wurde. Synergismus wird durch die Gabe von subtoxischen Konzentrationen von Salicylsäure, Methyl-DOPA, Ascorbinsäure, anti-CAT, anti-SOD, nicht aber Anti-EGFR (einem irrelevanten Antikörper) erreicht.

Dass diese Synergiewirkung zwischen subtoxischer Konzentration Katalasehemmstoff und einem NO-Modulator sich tatsächlich auf einem Synergieeffekt bei der Katalaseinaktivierung wiederspiegelt wird in Figur 21 gezeigt. In diesem Experiment erfolgt wiederum ein Peroxynitrit-Challenge nach Vorbehandlung mit bestimmten Wirkstoffen. Die danach einsetzende Apoptose zeigt das Ausmaß der Katalaseinaktivierung an. Es ist erkennbar, dass die gewählte Salicylsäurekonzentration allein fast keine Katalaseinaktivierung nach sich zieht, die Kombination von Salicylsäure mit Taxol jedoch dessen Wirkung drastisch erhöht. Dabei wird abgesichert, dass die Inaktivierung der Katalase eine gesicherte biochemische Konsequenz des hier vorgestellten Synergiesystems darstellt.

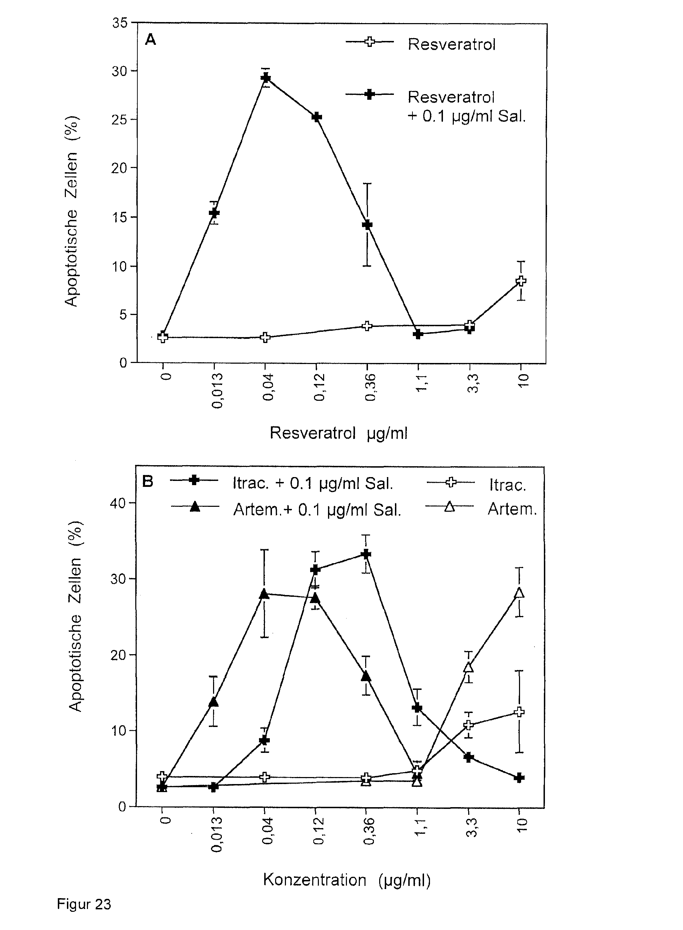

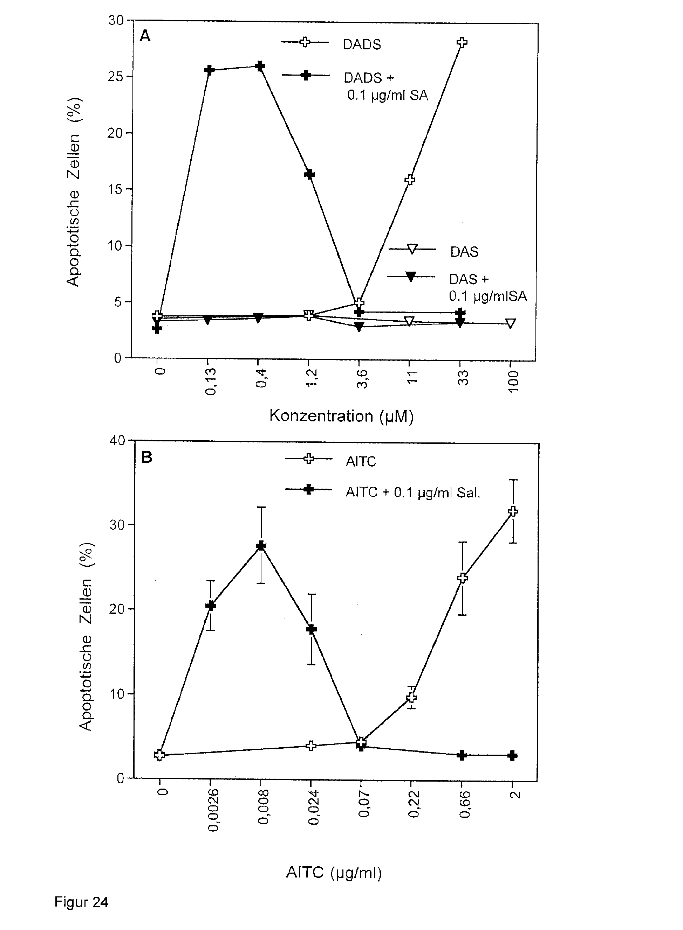

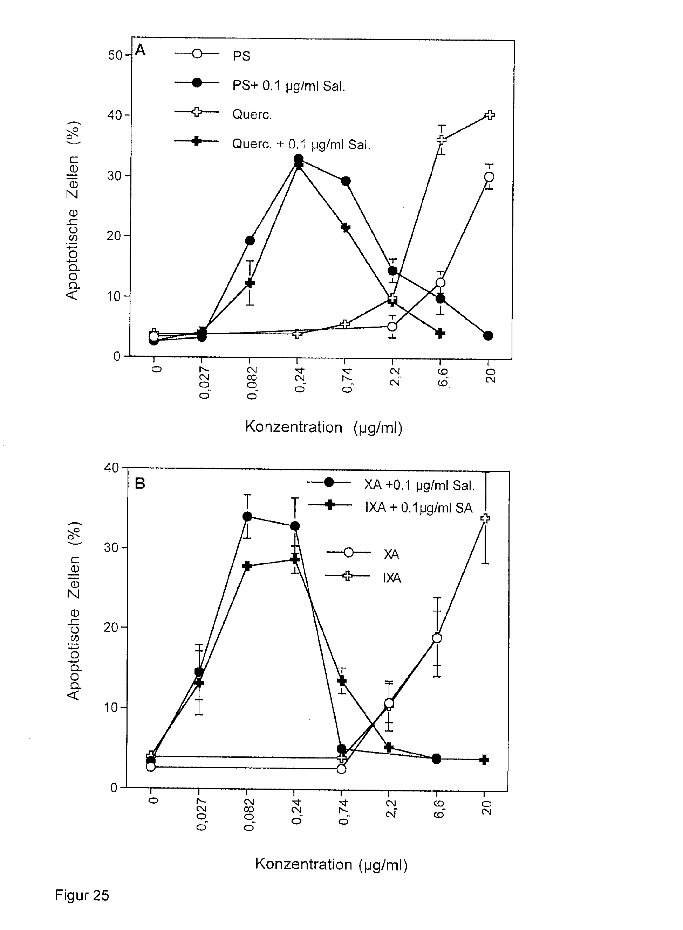

Die Allgemeingültigkeit der synergistischen Wirkung zwischen einem direkten Katalasehemmstoff (hier exemplarisch Salicylsäure) und Modulatoren der verschiedenen ROS-relevanten Signalwege wird in den nachfolgenden Figuren 22-25 dargestellt. Es zeigt sich Synergie zwischen Salicylsäure und Arginin oder Arginase (Abb. 17), zwei Stoffen, die direkt in die Verfügbarkeit von NO eingreifen, indem sie die NO-Synthase mit mehr Substrat versehen. Der Synergieeffekt zwischen Resveratrol, einem Stimulator der NADPH-Oxidase und Salicylsäure ist besonders eindrucksvoll (Figur 23A). Zum gewählten Beobachtungszeitpunkt führt die alleinige Gabe von Resveratrol noch zu keiner deutlichen Apoptoseinduktion, die Kombination mit einer subtoxischen Konzentration Salicylsäure erlaubt jedoch eine sehr starke Apoptoseinduktion. Synergieeffekte ergeben sich zwischen Salicylsäure und Hemmstoffen der NOD wie Itraconazol oder Artemisinin (Figur 23B), Diallyldisulfid (nicht jedoch Diallylsulfid) oder Allylisothiocyanat (Figur 24), Palmitinsäure, Quercetin, Xanthohumol und Isoxanthohumol (Figur 25).

### 1.3 Modellexperimente zur Klärung der unerwarteten synergistischen Wechselwirkung zwischen direkten Katalaseinhibitoren und Modulatoren des NO- bzw. Superoxidanionstoffwechsels, die zu einer Inaktivierung von Katalase führen

Aufgrund der Datenlage vor dieser Anmeldung durfte angenommen werden, dass sich parallel durchgeführte direkte Katalasehemmung und Inaktivierung der Katalase durch Singulettsauerstoff, der nach Modulation des NO- oder Superoxidanionenstoffwechsels entsteht, lediglich additiv in ihrer Wirkung verstärken sollten. So war auch nicht zu erwarten, dass die Anwesenheit einer geringer Konzentrationen eines direkten Katalasehemmstoffes, die für sich keine messbare Wirkung in dem untersuchten System zeigt, eine erkennbare Steigerung der Wirkung eines Modulators herbeiführt, der über die Bildung von Singulettsauerstoff Katalase inaktiviert. Die in dieser Anmeldung aufgeführten Beispiele zeigen jedoch das Gegenteil dieser Erwartung.

Da sich die Signalingprozesse auf der Membran einzelner Zellen nicht ohne weiteres analytisch auflösen lassen, wurde zur Klärung des zugrunde liegenden Sachverhalts ein Modellsystem eingesetzt, in dem ein definierter Anteil der Tumorzellpopulation seiner protektiven Katalase beraubt, der restliche Anteil der Population volle Protektion durch Katalase besitzt. Die Entfernung der protektiven Katalase wurde durch siRNA-vermittelten Knockdown der Katalase erreicht, wie in Heinzelmann and Bauer 2010 beschrieben.

Figuren 26-29 zeigen zunächst, dass die Reinpopulation von Kontroll-Tumorzellen erwartungsgemäß nicht in Apoptose geht, während die Reinpopulation Katalase-negativen Tumorzellen durch ROS-Signaling in die Apoptose getrieben wird. Wird ein kleiner Prozent Katalase-negativer Tumorzellen mit unbehandelten Tumorzellen gemischt, verhält sich diese Population nach einer gewissen zeitlichen Verzögerung so, als ob die Mehrzahl der Zellen keinen Schutz durch Katalase besitzen würde. Ausgehend von den wenigen Katalase-negativen Zellen muss es also zu einer Inaktivierung der Katalase der Nachbarzellen gekommen sein. Dieser "quasiinfektiöse" oder "Bystander"-Prozess wird, wie die Inhibitordaten zeigen, durch Singulettsauerstoff bewirkt, der sich wiederum von Peroxynitrit und Wasserstoffperoxid ableitet.

*Eine lokalisiert vorliegende Katalasehemmung beschleunigt also Singulettsauerstoffbildung und Katalaseinaktivierung in ihrer zellulären Umgebung.*

Es ist offensichtlich, dass dieses für die Interaktion von Zellen etablierte Modell auch auf einzelne Zellen übertragen werden kann, die gedanklich in Bereiche mit intakter und inaktivierter Katalase aufgetrennt werden können. Die in den Beispielen dieser Anmeldung gezeigte Synergiewirkung lässt sich somit folgendermaßen erklären:

Die Katalase der Tumorzellen verhindert durch effizienten Abbau von Wasserstoffperoxid und Peroxynitrit sowohl interzelluläres ROS-Signaling als auch Singulettsauerstoffbildung. Nach Steigerung der verfügbaren NO-Konzentration, z. B. durch einen NOD-Hemmer, kommt es transient zur Singulettsauerstoffbildung, die über FAS-Aktivierung zur nachfolgenden Steigerung der Superoxidanionenproduktion führt. Dieser Amplifikationsschritt schlägt sich in gesteigerter Singulettsauerstoffbildung nieder, so dass schließlich eine ausreichend große Zahl protektiver Katalasemoleküle inaktiviert werden und apoptotisches ROS-Signaling ablaufen kann. Bei Anwesenheit kleiner Konzentrationen eines direkten Katalasehemmstoffes wird die initiale Singulettsauerstoffbildung wesentlich erleichtert und dadurch die Inaktivierung der Katalase schneller erzielt. Die synergistisch wirksame geringe Konzentration des direkten Katalasehemmstoffes wirkt im übertragenen Sinn wie ein Brandbeschleuniger oder eine Glühkerze in einem Dieselmotor. Die Beschleunigung und Steigerung der Effizienz des Gesamtvorgangs wird insbesondere daran erkennbar, dass bei synergistischer Wirkung

zwischen einem direkten Katalasehemmstoff und z. B. einem NOD-Hemmstoff keine Verstärkung des Prozesses durch den FAS-Rezeptor / Caspase-8 mehr notwendig ist, während die Wirkung des NOD-Hemmstoffes allein dieser Verstärkung bedarf.

## Patentansprüche

1. Pharmazeutische Zusammensetzung, **dadurch gekennzeichnet, dass** sie wenigstens einen Wirkstoff enthält, der eine Singulettsauerstoff-unabhängige direkte Katalase-Inaktivierung bewirken kann und, dass die Zusammensetzung weiterhin mindestens einen Wirkstoff enthält, der durch Modulation des Stickoxid oder Superoxidanionenstoffwechsels der Zellen und nachfolgender Singulettsauerstoffbildung zur Inaktivierung von Katalase führt.

2. Pharmazeutische Zusammensetzung nach Anspruch 1, **dadurch gekennzeichnet, dass** der Wirkstoff, der zu Singulettsauerstoff-unabhängiger direkter Inaktivierung der Katalase fähig ist, ausgewählt ist aus der Gruppe bestehend aus Antikörpern gegen Katalase, Antikörpern gegen Superoxiddismutase, Salicylsäure, Ascorbinsäure und Methyl-dopa.

3. Pharmazeutische Zusammensetzung nach Anspruch 1 oder 2, **dadurch gekennzeichnet, dass** der Wirkstoff, der durch Modulation des Stickoxid- oder Superoxidanionenstoffwechsels der Zellen und nachfolgender Singulettsauerstoffbildung zur Inaktivierung von Katalase führt, ausgewählt ist aus einer Stoffgruppe, die die verfügbare Stickoxidkonzentration in Zellen erhöhen kann.

4. Pharmazeutische Zusammensetzung nach Anspruch 3, **dadurch gekennzeichnet, dass** der Wirkstoff, der durch Modulation des Stickoxid- oder Superoxidanionenstoffwechsels der Zellen und nachfolgender Singulettsauerstoffbildung zur Inaktivierung von Katalase führt, ausgewählt ist aus der Gruppe bestehend aus Flavonoiden, Anthocyanen, Taxol, Epothilon B, Artemisinin, Diallyldisulfid, Nor-NOHA, 2(S)-Amino-6-boronohexansäure NH⁺₄ (A-6-BHA), (2S)-(+)-Amino-5-iodoacetamidopentansäure (A-5-IAAPA), S-(2-Boronoethyl)-L-cysteine NH⁺₄ (BEC), N^{G}-Hydroxy-L-arginine.monoacetat (NOHA), Azolen, insbesondere Itraconazol, Econazol, Fluconazol, Mikonazol, Ketokonazol, Isothiocyanate, insbesondere Methylisothiocyanat, Allylisothiocyanat und Sulforaphan.

5. Pharmazeutische Zusammensetzung nach einem der Ansprüche 1 bis 3, **dadurch gekennzeichnet, dass** der Wirkstoff, der eine Singulettsauerstoff-unabhängige direkte Katalase-Inaktivierung bewirken kann, ausgewählt ist aus humanen oder humanisierten Antikörpern gegen membranständige humane Katalase oder humanen oder humanisierten Antikörpern gegen humane Superoxiddismutase und, dass der Wirkstoff, der durch Modulation des Stickoxid- oder Superoxidanionenstoffwechsels der Zellen und nachfolgender Singulettsauerstoffbildung zur Inaktivierung der Katalase führt, ausgewählt ist aus Taxol, einem Anthocyan und/oder Diallyldisulfid.

6. Pharmazeutische Zusammensetzung nach Anspruch 5, **dadurch gekennzeichnet, dass** der Wirkstoff, der eine Singulettsauerstoff-unabhängige direkte Katalase-Inaktivierung bewirken kann, ein humaner oder humanisierter Antikörper gegen membranständige humane Katalase ist und, dass der Wirkstoff, der durch Modulation des Stickoxid- oder Superoxidanionenstoffwechsels der Zellen und nachfolgender Singulettsauerstoffbildung zur Inaktivierung von Katalase führt, Taxol ist.

7. Pharmazeutische Zusammensetzung nach Anspruch 5, **dadurch gekennzeichnet, dass** der Wirkstoff, der eine Singulettsauerstoff-unabhängige direkte Katalase-Inaktivierung bewirken kann, ein humaner oder humanisierter Antikörper gegen membranständige humane Katalase ist und, dass der Wirkstoff, der durch Modulation des Stickoxid- oder Superoxidanionenstoffwechsels der Zellen und nachfolgender Singulettsauerstoffbildung zur Inaktivierung von Katalase führt, Anthocyan ist.

8. Pharmazeutische Zusammensetzung nach Anspruch 5, **dadurch gekennzeichnet, dass** der Wirkstoff, der eine Singulettsauerstoff-unabhängige direkte Katalase-Inaktivierung bewirken kann, ein humaner oder humanisierter Antikörper gegen membranständige humane Katalase ist und, dass der Wirkstoff, der durch Modulation des Stickoxid- oder Superoxidanionenstoffwechsels der Zellen und nachfolgender Singulettsauerstoffbildung zur Inaktivierung von Katalase führt, Diallyldisulfid ist.

9. Pharmazeutische Zusammensetzung nach einem der Ansprüche 4 bis 8, **dadurch gekennzeichnet, dass** die Zusammensetzung weiterhin Resveratrol umfasst.

10. Pharmazeutische Zusammensetzung nach einem der vorhergehenden Ansprüche zur Verwendung bei der Behandlung einer Tumorerkrankung.

11. Pharmazeutische Zusammensetzung nach Anspruch 10, **dadurch gekennzeichnet, dass** die Tumorerkrankung ausgewählt ist aus Magenkarzinom und/oder Lymphom.
